# EUROPEAN PATENT SPECIFICATION

(11) **EP 0 467 848 B1**
(45) Date of publication and mention of the grant of the patent: **31.01.1996**
(21) Application number: 91810559.4
(22) Date of filing: 11.07.1991
(51) Int. Cl.: C07C 69/54, C07C 57/075, C07C 15/46, C07C 67/62, C07C 7/20

(54) **Stabilized monomer compositions**
Stabilisierte Monomerenzusammensetzungen
Compositions monomères stabilisées

(30) Priority: 20.07.1990 US 556240
(43) Date of publication of application: 22.01.1992
(73) Proprietor: CIBA-GEIGY AG, CH-4002 Basel (CH)
(72) Inventor: Gatechair, Leslie R., Katonah, NY 10536 (US); Hyun, James L., Danbury, CT 06811 (US); Schirmann, Peter J., Fairfield, CT 06430 (US)

(56) References cited:
- DE-B- 2 113 246
- US-A- 4 668 721
- US-A- 4 691 015
- DIE MAKROMOLEKULARE CHEMIE, vol. 160, October 18,1972 Y. MIURA et al. "Vinyl Polymerization. 286. Inhibiting Effect of 2.2.6.6-Tetramethyl -4-oxo- piperidine-1-oxyl in Radical Polymerization" pages 243-249

## Description

The instant invention pertains to stabilized monomer compositions, in particular to compositions containing ethylenically unsaturated monomers, stabilized against premature polymerization.

The ethylenically unsaturated compounds which can be polymerized by free radical initiation are commonly called monomers. They constitute a major class of industrial chemicals. Because of the presence of the polymerizable double bond, the widespread sources of initiating radicals from peroxides, light and/or thermal generation, such monomers are prone to undesirable and premature polymerization at various stages during their manufacture, purification, storage, shipping, blending and use. Protection of such monomers from such premature polymerization is needed up to the point where polymerization is actually desired. If premature polymerization does occur, the monomer may suffer contamination by polymer, troublesome increase in viscosity, gelation and/or loss of reactivity. Fouling of distillation equipment including heat exchanger surfaces, storage vessels, transfer lines, pumps, shipping containers and application equipment can occur with ensuing costs of cleaning, downtime, loss of material and unnecessary labor costs. A particularly difficult situation is the preparation of polyol acrylates from polyols and acrylic acid since prolonged heating periods are required to complete the esterification. Premature polymerization can also constitute a safety hazard since uncontrolled exothermic polymerization can cause ruptured vessels, atmospheric contamination, and in extreme cases, explosions and fires. Deterioration of monomers in shipping and storage may also make necessary the use of costly refrigerated shipping and storage facilities.

A further problem is that of undesired polymerization of adventitious monomers, that is, radically-polymerizable unsaturated monomers which occur in commercial products such as hydrocarbon fuels and refinery streams. In these cases, polymerization accompanied by the incorporation of oxygen moieties leads to gum and sludge deposits which can foul carburators, engines, fuel tanks or fuel lines. In refineries, the adventitious monomers in hydrocarbon streams such as cracking products can foul pipelines, valves, pumps, heat exchangers, stills and storage vessels.

Another problem in regard to undesired polymerization of free radical polymerizable monomers is the case of polymerizations which are intentional, but which must be prevented from going too far. For example, the quality of poly(vinyl chloride) suspension polymer and of synthetic rubber made from olefins and dienes is superior (i.e. better molecular weight distribution, stability, and processing properties) if the polymerization is stopped short of complete consumption of the monomers. It is also desirable to have available in a plant conducting vinyl polymerization reactions some rapid and efficient means for stopping a runaway polymerization if other means such as cooling should fail.

It is known that the addition of certain compounds to monomers can retard or even prevent their undesired polymerization, and that when polymerization of the monomer is desired, the inhibitor can be removed or overridden by a deliberately-added polymerization initiator. Various aromatic compounds have been used as such inhibitors in the prior art. Typical ones are hydroquinone, monomethyl ether of hydroquinone (MEHQ), tert-butylphenols, phenothiazine, phenylenediamines and benzoquinones. These are usually used at a level of 50 to 1000 ppm. These inhibitors are not totally effective and even with such inhibitors present, it is often advisable to store such inhibited monomers in a cool place and for limited periods of time. Moreover, these aromatic inhibitors are a cause of serious discoloration problems in the monomers and in polymers deliberately prepared from such monomers. Typically these aromatic inhibitors produce quinoidal chromophoric groups with very high visible light absorbance. The use of stable nitroxyl radicals as inhibitors also leads to discoloration since such compounds are themselves highly colored, usually bright red.

In order to overcome these color problems, a diligent search was made to find alternative inhibitors which are both effective and not discoloring. This search led to the N,N-dialkylhydroxylamines and the N,N-diaralkylhydroxylamines. Some typical references are cited infra.

US-A-3,222,334 and US-A-3,878,181 disclose the use of N,N-dialkylhydroxylamines such as N,N-diethylhydroxylamine as short-stopping agents for emulsion polymerizations of butadiene/styrene rubber and chloroprene.
DE-21 13 246 describes the use of cyclic hindered amines or their 1-hydroxy derivatives as vinyl polymerization inhibitors.

US-A-3,148,225 and US-A-3,697,470 disclose the use of N,N-dialkylhydroxylamines such as N,N-diethylhydroxylamine and N-alkyl-N-arylhydroxylamine such as N-ethyl-N-phenylhydroxylamine respectively as short-stopping agents and popcorn polymer inhibitors in processes for preparing synthetic rubber. The popcorn polymer formation is a serious problem encountered in recovering of monomers from such synthetic rubber operations.

US-A-4,782,105 teaches the use of long chain N,N-dialkylhydroxylamines as stabilizers to prevent the premature gelation of unsaturated elastomer compositions such as styrene/butadiene copolymers or polybutadiene.

US-A-3,408,422 describes the use of N,N-dialkylhydroxylamines such as N,N-diethylhydroxylamine and N,N-diaralkylhydroxylamines such as N,N-dibenzylhydroxylamine as stabilizers for preventing the premature gelation of unsaturated polyesters.

US-A-4,798,889 teaches the use of N,N-dialkylhydroxylamines such as N,N-diethylhydroxylamine or N,N-dibenzylhydroxylamine as stabilizers to reduce the thermal polymerization of organosiloxanes substituted by ethylenically unsaturated moieties.

US-A-4,409,408 and US-A-4,434,307 disclose the use of N,N-dibenzylhydroxylamine in combination with an alkylated diphenol (catechol or hydroquinone) as inhibitors to prevent the polymerization of styrene.

The use of stable nitroxyl radicals including those derived from hindered amine moieties has also been disclosed. Typical references are cited below.

SU-A-1,139,722 describes the inhibition of styrene and comonomers such as butadiene using 1-oxyl derivatives of hindered amine compounds such as N,N'-bis(1-oxyl-2,2,6,6-tetramethylpiperidin-4-yl)adipamide. The elimination of popcorn polymer and of the clogging of equipment is touted as the result of using such 1-oxyl compounds.

JP-A-60-36501 describes the use of hindered amines and their 1-oxyl and 1-alkyl derivatives as vinyl polymerization inhibitors to improve storage stability of monomers such as acrylate and methacrylate esters.

EP-A-178,168 and GB-A-1,127,127 describe the use of 1-oxyl substituted hindered amine compounds as stabilizers for inhibiting the polymerization of α,β-ethylenically unsaturated monocarboxylic acids, such as acrylic acid, during its recovery by distillation.

US-A-4,670,131 teaches the use of 1-oxyl substituted hindered amine compounds as stabilizers for preventing the fouling of equipment for processing organic feed streams containing olefins by inhibiting the polymerization of said olefins.

In a theoretical study of the inhibiting effects of selected hindered amine compounds, Y. Miura et al., Makromol. Chem. 160, 243 (1972) disclose that 1-oxyl-2,2,6,6-tetramethylpiperidin-4-one is highly effective in retarding the onset of the polymerization of styrene and methyl methacrylate. By contrast, the corresponding 1-hydroxy-2,2,6,6-tetramethylpiperidin-4-one is stated to have no effect in delaying the polymerization of styrene and only a weak retarding effect on said polymerization once begun.

US-A-4,668,721 and US-A-4,691,015 disclose the use of 1-hydroxy substituted hindered amine compounds as stabilizers for polyolefin compositions in combination with one or more other stabilizers such as phenolic antioxidants, ultraviolet light absorbers and the like.

None of these references describes or suggests that 1-hydroxy substituted hindered amine compounds are or could possibly be effective inhibitors to prevent the premature polymerization of monomers in either the liquid or vapor phase.

It is the broad object of the invention to provide monomer compositions inhibited against undesired and premature polymerization by means of small, but effective amounts of selected additives which do not impart undesired color to the monomer compositions.

It is a further object of the invention to provide inhibited monomer compositions which have substantially improved stability relative to compositions inhibited by methods known in the prior art.

It is a further object of the invention to provide a means for short-stopping or retarding polymerization of monomers once polymerization is started.

It is a further object of the invention to provide effective inhibitors for monomers known to be difficult to inhibit such as acrylic acid.

It still a further object of the invention to provide highly effective combinations of inhibitors for said monomers.

The invention pertains to a monomer composition, stabilized against premature polymerization, which comprises
(a) an ethylenically unsaturated monomer or mixture of monomers, polymerizable by free radical initiation, and
(b) an effective amount, sufficient to inhibit the premature polymerization of component (a), of a compound or mixture of compounds of any of formula I to XIII, and salts thereof,

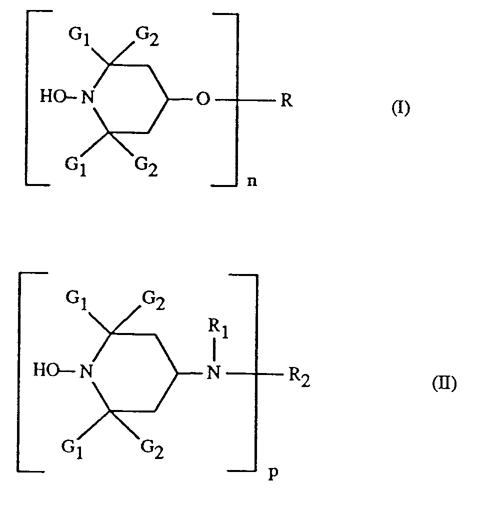

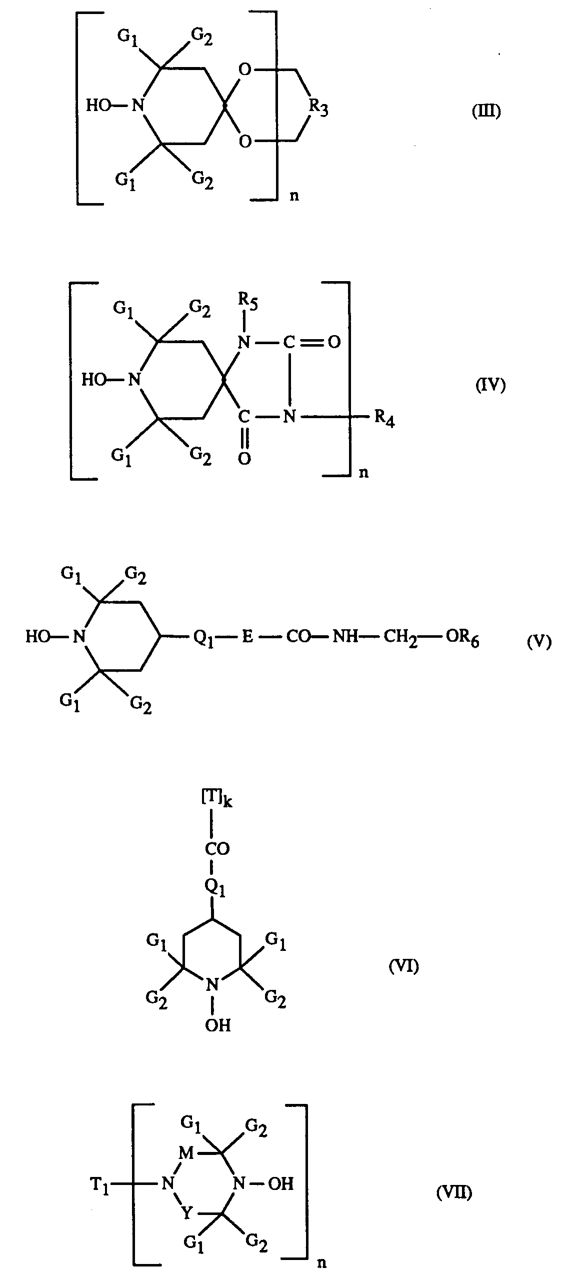

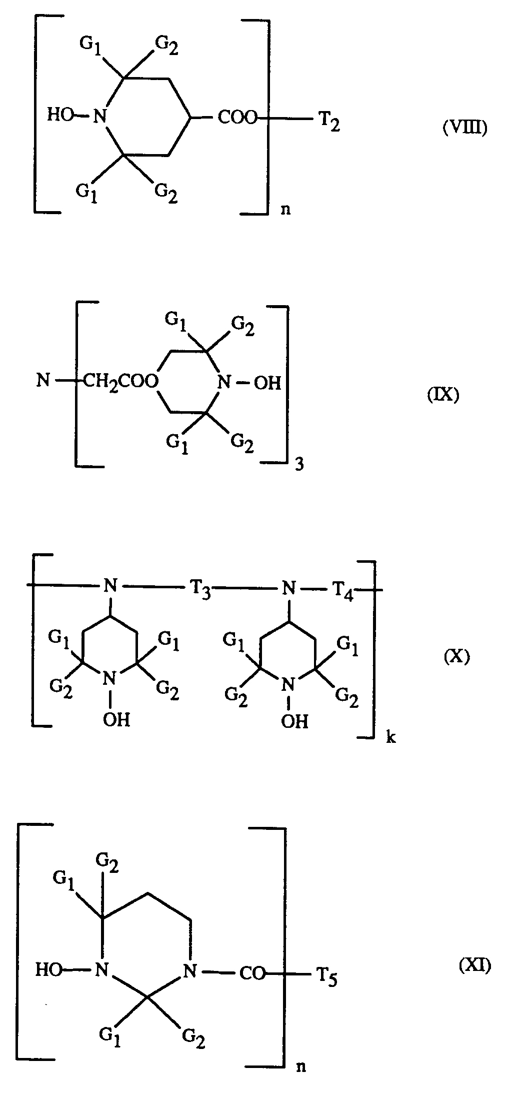

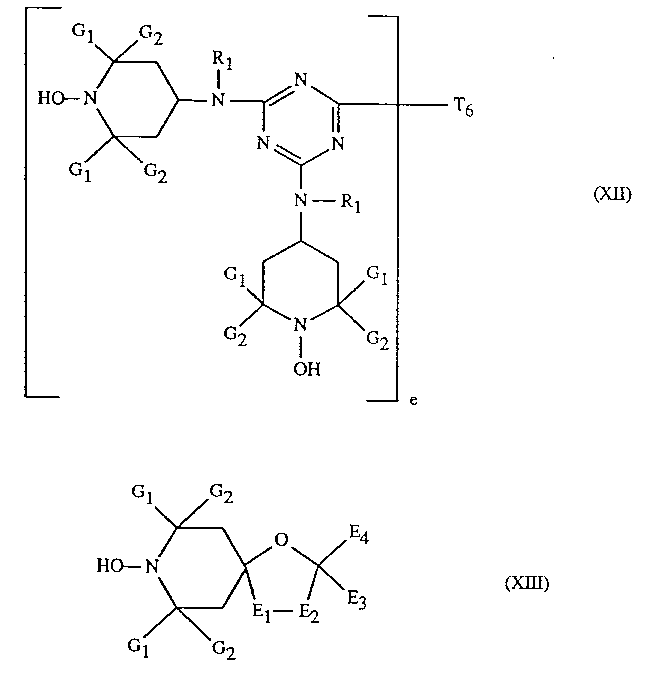

wherein
G₁ and G₂ are independently alkyl of 1 to 4 carbon atoms, preferably methyl, or G₁ and G₂ together are pentamethylene;
n is 1 or 2,
when n is 1,
R is C₁-C₁₈-alkyl optionally interrupted by one or more oxygen atoms, cyanoethyl, benzyl, glycidyl, a monovalent acyl radical of an aliphatic, cycloaliphatic, araliphatic or aromatic acid, or of carbamic acid or of a phosphorus-containing acid, or a monovalent silyl radical, preferably an acyl radical of an aliphatic carboxylic acid having 2 to 18 carbon atoms, of a cycloaliphatic acid having 5 to 12 carbon atoms or of an aromatic carboxylic acid having 7 to 15 carbon atoms, or of carbamic acid; or
when n is 2,
R is C₁-C₁₂-alkylene, C₄-C₁₂-alkenylene, xylylene, a divalent acyl radical of an aliphatic, cycloaliphatic, araliphatic or aromatic dicarboxylic acid, or of a dicarbamic acid or of a phosphorus-containing acid, or a bivalent silyl radical, preferably an acyl radical of an aliphatic dicarboxylic acid having 2 to 36 carbon atoms, of a cycloaliphatic or aromatic dicarboxylic acid having 8 to 14 carbon atoms, or of a aromatic dicarbamic acid having 8 to 14 carbon atoms;
p is 1, 2 or 3,
R₁ is hydrogen, C₁-C₁₂-alkyl, C₅-C₇-cycloalkyl, C₇-C₈-aralkyl, C₂-C₁₈-alkanoyl, C₃-C₅-alkenoyl or benzoyl;
when p is 1,
R₂ is hydrogen, C₁-C₁₈-alkyl, C₅-C₇-cycloalkyl, C₂-C₈-alkenyl unsubstituted or substituted by a cyano, carbonyl or carbamide group, or is glycidyl, a group of the formula -CH₂CH(OH)-Z or of the formula -CONH-Z wherein Z is hydrogen, methyl or phenyl; or
when p is 2,
R₂ is C₂-C₁₂-alkylene, C₆-C₁₂-arylene, xylylene, a -CH₂CH(OH)CH₂-O-X-O-CH₂CH(OH)CH₂- wherein X is C₂-C₁₀-alkylene, C₆-C₁₅-arylene or C₆-C₁₂-cycloalkylene; or, provided that R₁ is not alkanoyl, alkenoyl or benzoyl, R₂ can also be a divalent acyl radical of an aliphatic, cycloaliphatic or aromatic dicarboxylic acid or dicarbamic acid, or can be the group -CO-; or R₁ and R₂ together when p is 1 can be the cyclic acyl radical of an aliphatic or aromatic 1,2-or 1,3-dicarboxylic acid; or
R₂ is 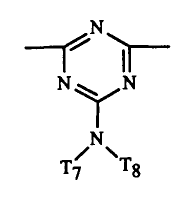
where T₇ and T₈ are independently hydrogen, alkyl of 1 to 18 carbon atoms, or T₇ and T₈ together are alkylene of 4 to 6 carbon atoms or 3-oxapentamethylene, preferably T₇ and T₈ are 3-oxapentamethylene;
when p is 3,
R₂ is 2,4,6-triazinyl;
when n is 1,
R₃ is C₂-C₈-alkylene or hydroxyalkylene or C₄-C₂₂-acyloxyalkylene; or
when n is 2,
R₃ is (-CH₂)₂C(CH₂-)₂;
when n is 1,
R₄ is hydrogen, C₁-C₁₂-alkyl, C₃-C₅-alkenyl, C₇-C₉-aralkyl, C₅-C₇-cycloalkyl, C₂-C₄-hydroxyalkyl, C₂-C₆-alkoxyalkyl, C₆-C₁₀-aryl, glycidyl, a group of formula -(CH₂)ₘ-COO-Q or of the formula (CH₂)ₘ-O-CO-Q wherein m is 1 or 2 and Q is C₁-C₄-alkyl or phenyl; or
when n is 2,
R₄ is C₂-C₁₂-alkylene, C₆-C₁₂-arylene, a group -CH₂CH(OH)CH₂-O-X-O-CH₂CH(OH)CH₂- wherein X is C₂-C₁₀-alkylene, C₆-C₁₅-arylene or C₆-C₁₂-cycloalkylene, or a group -CH₂CH(OZ₁)CH₂-(OCH₂CH(OZ₁)CH₂)₂- wherein Z₁ is hydrogen, C₁-C₁₈-alkyl, allyl, benzyl, C₂-C₁₂-alkanoyl or benzoyl;
R₅ is hydrogen, C₁-C₁₂-alkyl, allyl, benzyl, glycidyl or C₂-C₆-alkoxyalkyl;
Q₁ is -N(R₇)- or -O-;
E is C₁-C₃-alkylene, the group -CH₂CH(R₈)-O- wherein R₈ is hydrogen, methyl or phenyl, the group -(CH₂)₃-NH- or a direct bond;
R₇ is hydrogen, C₁-C₁₈-alkyl, C₅-C₇-cycloalkyl, C₇-C₁₂-aralkyl, cyanoethyl, C₆-C₁₀-aryl, the group -CH₂CH(R₈)-OH; or a group of the formula 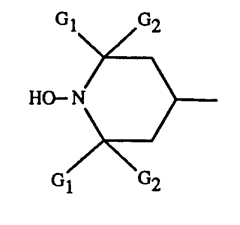
or a group of the formula 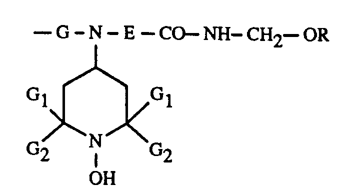
wherein G is C₂-C₆-alkylene or C₆-C₁₂-arylene; or
R₇ is a group -E-CO-NH-CH₂-OR₆;
R₆ is hydrogen or C₁-C₁₈-alkyl;
Formula VI denotes a recurring structural unit of a polymer where T is ethylene or 1,2-propylene, or is a repeating structural unit derived from an α-olefin copolymer with an alkyl acrylate or methacrylate, preferably a copolymer of ethylene and ethyl acrylate;
k is 2 to 100;
T₁ has the same meaning as R₂ when p is 1 or 2;
M and Y are independently methylene or carbonyl, preferably M is methylene and Y is carbonyl, and T₁ is ethylene when n is 2;
T₂ has the same meaning as R₄, and T₂ is preferably octamethylene when n is 2,
T₃ and T₄ are independently alkylene of 2 to 12 carbon atoms, or T₄ is 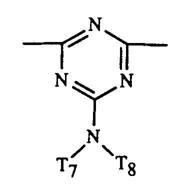
T₆ is 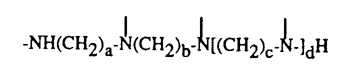
where a, b and c are independently 2 or 3, and d is 0 or 1, preferably a and c are each 3, b is 2 and d is 1;
e is 3 or 4, preferably 4;
T₅ is the same as R with the proviso that T₅ cannot be hydrogen when n is 1;
E₁ and E₂, being different, are each oxo or imino, preferably E₁ is oxo and E₂ is -N(E₅)- where E₅ is hydrogen, C₁-C₁₂-alkyl or alkoxycarbonylalkyl of 4 to 22 carbon atoms;
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms; and
E₄ is hydrogen, alkyl of 1 to 30 carbon atoms, phenyl, naphthyl or phenylalkyl of 7 to 12 carbon atoms; or
E₃ and E₄ together are polymethylene of 4 to 17 carbon atoms, or said polymethylene substituted by up to four alkyl groups of 1 to 4 carbon atoms, preferably methyl.

The monomers of component (a) of this invention are any having at least one carbon-carbon double bond capable of undergoing free radical induced polymerization. Such monomers are well known in commerce and comprise a wide variety of structural types. Typical examples of such monomers are the olefinic hydrocarbons such as styrene, α-methylstyrene and divinylbenzene; dienes such as butadiene and isoprene; halogenated monomers such as vinyl chloride, chloroprene, vinylidene chloride, vinylidene fluoride and vinyl fluoride; unsaturated acids such as acrylic acid, methacrylic acid and crotonic acid; unsaturated esters such as vinyl acetate, alkyl acrylates and alkyl methacrylates such as methyl methacrylate, ethyl acrylate, methyl acrylate, 2-hydroxyethyl acrylate and methacrylate, ethylene bismethacrylate, trimethylolpropane triacrylate, acrylated epoxy resin and polyethylene glycol diacrylate; unsaturated amides such as acrylamide, N,N-dimethylacrylamide, methylene-bisacrylamide and N-vinylpyrrolidone; unsaturated nitrile monomers such as acrylonitrile; and unsaturated ethers such as methyl vinyl ether; and miscellaneous monomers such as the vinyl pyridines, diethyl vinylphosphonate and sodium styrenesulfonate.

The instant invention also pertains to the use of mixtures of said monomers and to the use of resins such as acrylate-terminated polyurethanes and unsaturated polyesters. The common feature making all of these materials relevant to the present invention is the presence of a polymerizable double bond.

Also in the category of monomers are unsaturated oils such as drying oils like linseed oil, where polymerization also incorporates oxygen. There are also adventitious monomers formed in refining processes, for example polymerizable olefinic unsaturation in gasoline, jet fuel, solvents, crude oil and cracked hydrocarbon streams. The common feature of all of these substances is encompassed in the broad term "monomers" and all are contemplated to be within the scope of instant component (a). Polymerization of such materials is often accompanied by autooxidation.

The acrylates, particularly acrylic acid itself, are unusually difficult to inhibit beacuse of their inherent high polymerizability. The instant compounds are shown to be particularly effective in inhibiting acrylic acid from premature polymerization.

Preferably component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

Most preferably the monomer of component (a) is styrene, butadiene, vinyl chloride, acrylic acid, methacrylic acid, vinyl acetate, 2-hydroxyethyl acrylate, 2-hydroxyethyl methacrylate, trimethylolpropane triacrylate, polyethylene glycol diacrylate or methyl methacrylate.

Still more preferably the monomer is styrene, butadiene, acrylic acid or methacrylic acid.

The hydroxylamine derivatives useful in the instant invention are denoted by the various structures of formulas I to XIII. Most of these hydroxylamine derivatives are known compounds. The instant hydroxylamine derivatives can be easily prepared from the corresponding hindered amine many of which are commerically available or which can be made by known procedures. Particularly preferred derivatives are those of formula I, II and VII, preferably of formula I. Further preferred derivatives are
N-(1-hydroxy-2,2,6,6-tetramethylpiperidin-4-yl) caprolactam;
bis(1-hydroxy-2,2,6,6-tetramethylpipendin-4-yl) sebacate;
1-hydroxy-2,2,6,6-tetramethylpiperidin-4-yl benzoate;
1-hydroxy-2,2,6,6-tetramethylpiperidin-4-yl acrylate;
1-hydroxy-2,2,6,6-tetramethylpiperidin-4-yl 3,5-di-tert-butyl-4-hydroxybenzoate;
4,4'-ethylenebis(1-hydroxy-2,2,6,6-tetramethylpiperazin-3-one);
1-hydroxy-2,2,6,6-tetramethylpiperidin-4-yl stearate;
bis(1-hydroxy-2,2,6,6-tetramethylpiperidin-4-yl) o-phthalate;
bis(1-hydroxy-2,2,6,6-tetramethylpiperidin-4-yl) 3,5-di-tertbutyl-4-hydroxybenzylbutyl-malonate;
bis(1-hydroxy-2,2,6,6-tetramethylpiperidin-4-yl) n-butylmalonate;
2,10-di-tert-butyl-4,8-dimethyl-6-(1-hydroxy-2,2,6,6-tetramethyl-piperidin-4-yloxy)dibenzo[d,g][1,3,2]dioxaphosphocin;
1-hydroxy-2,2,6,6-tetramethylpiperidin-4-yl 2-ethylhexanoate;
dinonylnaphthalene disulfonic salt of bis(1-hydroxy-2,2,6,6-tetramethylpiperidin-4-yl) sebacate; and
the phosphorus acid salt of bis(1-hydroxy-2,2,6,6-tetramethylpiperidin-4-yl) sebacate.

The hydroxylamine derivative may generally be prepared by oxidizing a hindered amine with a peroxy compound such as hydrogen peroxide followed by reduction of the oxyl intermediate formed to the desired hydroxylamine derivative. Such a process is taught in U.S. Patent No. 4,665,185.

If any of the substitutents are C₁-C₁₂-alkyl, they are for example methyl, ethyl, n-propyl, isopropyl, n-butyl, sec-butyl, tert-butyl, n-amyl, tert-amyl, n-hexyl, n-octyl, 2-ethylhexyl, tert-octyl, n-nonyl, n-decyl, n-undecyl or n-dodecyl. As C₁-C₁₈-alkyl, R can be the aforementioned groups, and in addition for example n-tridecyl, n-tetradecyl, n-hexadecyl or n-octadecyl.

If R is a monovalent acyl radical of a carboxylic acid, it is for example an acyl radical of acetic acid, stearic acid, salicylic acid, methacrylic acid, acrylic acid, maleic acid, benzoic acid, 2-ethylhexanoic acid or 3,5-di-tert-butyl-4-hydroxyhydrocinnamic acid.

If R is a divalent acyl radical of a dicarboxylic acid, it is for example an acyl radical of adipic acid, succinic acid, suberic acid, sebacic acid, o-phthalic acid, butylmalonic acid, dibutylmalonic acid, dibenzylmalonic acid, 3,5-di-tert-butyl-4-hydroxybenzylbutylmalonic acid or bicycloheptene dicarboxylic acid.

If R is a divalent acyl radical of a dicarbamic acid, it is for example an acyl radical of hexamethylenedicarbamic acid or 2,4-toluylenedicarbamic acid.

R is also an acyl radical of a phosphorus-containing acid of the formula 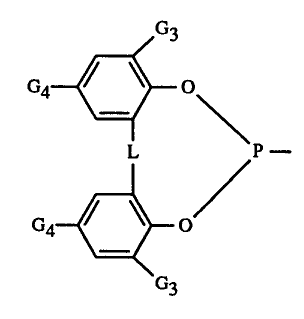
wherein L is a direct bond, methylene or alkylidene of 2 to 6 carbon atoms such as ethylidene, butylidene or amylidene. Preferably L is a direct bond, methylene or ethylidene.

G₃ and G₄ are independently alkyl of 1 to 4 carbon atoms, preferably methyl or tert-butyl. Most preferably G₃ and G₄ are each tert-butyl, or G₃ is tert-butyl and G₄ is methyl.

If any substituents are C₅-C₇-cycloalkyl, they are in particular cyclohexyl.

As C₇-C₈-aralkyl, R₁ is phenethyl and especially benzyl.

As C₂-C₁₈-alkanoyl, R₁ is for example propionyl, butyryl, octanoyl, lauroyl, hexadecanoyl, octadecanoyl, but especially acetyl; and as C₃-C₅-alkenoyl, R₁ is in particular acryloyl.

If R₂ is C₂-C₈-alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

When R₁ and R₂ are together a cyclic acyl radical, they are especially - CO-(CH₂)₅-.

If any substituents are C₂-C₁₂-alkylene, they are for example ethylene, propylene, 2,2,-dimethylpropylene, tetramethylene, hexamethylene, octamethylene, decamethylene or dodecamethylene.

If any substituents are C₆-C₁₅-arylene, they are for example o-, m- or p-phenylene, 1,4-naphthylene or 4,4'-diphenylene.

As C₆-C₁₂-cycloalkylene, X is especially cyclohexylene.

If R₃ is C₂-C₈-alkylene or hydroxyalkylene, it is for example ethylene, 1-methyl-ethylene, propylene, 2-ethylpropylene or 2-ethyl-2-hydroxymethylpropylene.

As C₄-C₂₂acyloxyalkylene, R₃ is for example 2-ethyl-2-acetoxymethyl-propylene.

If any substituents are C₂-C₆-alkoxyalkyl, they are example methoxymethyl, ethoxymethyl, propoxymethyl, tert-butoxymethyl, ethoxyethyl, ethoxypropyl, n-butoxyethyl, tert-butoxyethyl, isopropoxyethyl or propoxypropyl.

If R₄ is C₃-C₅-alkenyl, it is for example 1-propenyl, allyl, methallyl, 2-butenyl or 2-pentenyl.

As C₇-C₉-aralkyl, R₄ is phenethyl or especially benzyl; and as C₅-C₇-cyclohexyl is especially cyclohexyl.

If R₄ is C₂-C₄-hydroxyalkyl, it is for example 2-hydroxyethyl, 2-hydroxypropyl, 3-hydroxypropyl, 2-hydroxybutyl or 4-hydroxybutyl.

As C₆-C₁₀-aryl, R₄ is in particular phenyl or α- or β-naphthyl which is unsubstituted or substituted by halogen or C₁-C₄-alkyl.

If R₄ is C₂-C₁₂-alkylene, it is for example ethylene, propylene, 2,2-dimethylpropylene, tetramethylene, hexamethylene, octamethylene, decamethylene or dodecamethylene.

If R₄ is C₆-C₁₂-arylene, it is for example o-, m- or p-phenylene, 1,4-naphthylene or 4,4'-diphenylene.

If Z₁ is C₂-C₁₂-alkanoyl, it is for example propionyl, butyryl, octanoyl, dodecanoyl or preferably acetyl.

As C₅-C₇-cycloalkyl, R₇ is particularly cyclohexyl.

As C₆-C₁₀-aryl, R₇ is particularly phenyl or α- or β-naphthyl which is unsubstituted or substituted with halogen or C₁-C₄-alkyl.

As C₁-C₃-alkylene, E is for example methylene, ethylene or propylene.

As C₂-C₆-alkylene, G is for example ethylene, propylene, 2,2-dimethylpropylene, tetramethylene or hexamethylene; and as C₆-C₁₂-arylene, G is o-, m- or p-phenylene, 1,4-naphthylene or 4,4'-diphenylene.

An effective inhibiting amount of an instant compound of this invention needed to retard or prevent premature free radical induced polymerization of a monomer or monomer mixture is preferably in the range of from 1 to 10000 ppm, based on the total monomer composition, with the preferred range being 5 to 2000 ppm and the most preferred range being 50 to 1000 ppm. The lower amounts would be used where the degree of inhibition required is not great such as when the monomers are to be used promptly, or which will be stored refrigerated, or which are inherently less prone to polymerize readily such as monomers with internal double bonds. The higher amounts of inhibitor would be used where the monomer is to be stored for prolonged periods of time, especially under relatively warm conditions or where contamination is likely, or where exposure to photoinitiation is likely, or where the monomer is especially prone to rapid polymerization with little provocation such as with the acrylates and acrylic acid. Those skilled in the art of vinyl polymerization are well aware of the relative polymerizability of monomers and of their relative stabilities.

The stabilized compositions of this invention are distinguished by their lack of color.

The compositions of the instant invention may also contain additional inhibitors, such as hydroquinone, the monomethyl ether of hydroquinone, phenothiazine (these three often being required by monomer specifications) or catechol, tert-butylated hydroquinones or catechols, other alkylated phenols, nitrosophenols, nitrosophenylhydroxylamines, alkylated phenothiazines, sulfur and hindered cyclic amines or their corresponding oxyl derivatives.

The inhibited compositions may also contain metal deactivators and UV absorbers to improve light stability; or stabilizers such as amines to retard acid-catalyzed degradation; or thermal or photoinitiators; and other conventional additives.

When it is desired to subject the inhibited monomer to polymerization, the inhibitor can either be removed or overridden by sufficient polymerization initiator. Removal can be accomplished by distillation, absorption or washing with an acidic solution. It is possible to remove the instant 1-hydroxy derivatives while leaving the phenolic antioxidants in the monomer by use of acid ion exchange resins. The polymerization inhibiting action of the instant compounds can be overridden by use of sufficient free radical initiator, actinic light irradiation, electron beam exposure or other polymerization initiating means.
The instant invention also pertains to a process for preventing the premature polymerization of a monomer polymerizable by free radical initiation which comprises
adding to said monomer (a) an effective amount of a compound of component (b) as described above. The process of the instant invention involves simply dissolving an effective inhibiting amount of the inhibitor in the monomer prior to exposure of the latter to conditions where the premature, undesired free radical initiated polymerization might occur.

Preferably, this process comprises
adding 10 to 500 ppm of at least one compound of formula I to XIII to a continuous fluid feed stream to deactivate the autocatalytic polymerization, in any part of the continuous process equipment, such as reactor, reboiler, distillation column, etc., of any ethylenically unsaturated monomer present in the feed stream, and
further adding to said feed stream an additional 10 ppb to 500 ppm of at least one compound of formula I to XIII as a makeup additive to maintain the desired concentration of said compound in the fluid feed stream being processed.

Preferably, this process is also carried out to prevent the fouling of processing equipment including reactors, pipes, stills, distillation columns, cracking towers and heat transfer surfaces during the processing of a monomer polymerizable by free radical intiation.

The following examples are presented for the purpose of illustration only and are not to be construed as limiting the instant invention in any manner whatsoever.

### Example 1

Trimethylolpropane triacrylate is extracted with cold dilute aqueous alkali to remove the monomethyl ether of hydroquinone which is present as an inhibitor. To 25 g samples of the uninhibited monomer is added 20 ppm of the test inhibitor and the sample is then placed in a 28 ml amber bottle in an oven kept at 100°C. The time required for polymerization as visually observed by the formation of gelled lumps or solid matter to occur is a measure of the effectiveness of the test inhibitor.

| Inhibitor* | Hours to Failure (= Gelation) |
|---|---|
| None | 36 |
| Compound A | 211 |
| Compound B | 180 |

| | |
|---|---|
| *Compound A is N-(1-hydroxy-2,2,6,6-tetramethylpiperidin-4-yl)caprolactam; Compound B is Bis(1-hydroxy-2,2,6,6-tetramethylpiperidin-4-yl) sebacate. | |

### Example 2

The effect of the presence of the test inhibitor on the yellowness of a cured acrylic resin is determined by measuring yellowness index (YI). To a mixture of 50% by weight of epoxy acrylate (CELRAD® 3700, Celanese), 19% by weight of trimethylolpropane triacrylate, 19% by weight of ethoxylated trimethylolpropane triacrylate, 10% by weight of polyethylene glycol (200) diacrylate and 2% by weight of hydroxycyclohexyl phenyl ketone photoinitiator is added 20 ppm of the test inhibitor. The mixture is coated onto paper and passed at 10 ft/min (3.05 m/min) under a 200 watt/in medium pressure mercury arc lamp in a PPG UV Processor at full power.

The cure is evaluated by a standard surface hardness test method and the presence of the test inhibitor is found to have no adverse effect on the rate of cure or the hardness of the cured resin.

The yellowness index is then determined by means of an XL-10A Colorimeter (Gardner Laboratory) using a normal beam and a 3.8 cm orifice. A YI value is obtained immediately after curing is complete and then again after the cured resin is exposed to an accelerated weathering device for 24 hours to determine the light stability of the cured resin in a simulated office environment.

| Inhibitor* | Yellowness Index | |
|---|---|---|
| | After Cure | After Weathering |
| None | 4.2 | 14 |
| Compound A | 4.3 | 11.6 |
| Compound B | 4.6 | 11.5 |

| | | |
|---|---|---|
| * Compound A and Compound B are named in Example 1. | | |

These data indicate that the presence of the test inhibitor improves the resistance of the cured resin to yellowing under simulated office lighting conditions.

### Example 3

### Liquid Phase Inhibition

Test inhibitors are added at the 10 ppm and at the 100 ppm level into a variety of unihibited monomers. The monomer containing the test inhibitor is then held at 80°C in sealed bottles till polymerization of the monomer is observed visually. The time in hours till polymerization occurs is a measure of the effectiveness of the test compound as an inhibitor.

| Inhibitor* (ppm) | Hours to Failure (= Gelation) | | | | | |
|---|---|---|---|---|---|---|
| | Styr | HEMA | MMA | AA | HEA | VAc (at 60°C) |
| None | 48 | 55 | 81 | 48 | 304 | 330 |
| Compound A (10) | 48 | 90 | 120 | 164 | 2100 | 356 |
| Compound A(100) | 90 | 164 | 352 | 1000 | >10000 | >1100 |
| Compound B (10) | 48 | 90 | 128 | 250 | 1984 | 352 |
| Compound B(100) | 90 | 90 | 7150 | 1200 | 7700 | >1100 |
| Styr is styrene. HEMA is 2-hydroxyethyl methacrylate. MMA is methyl methacrylate. AA is acrylic acid. HEA is 2-hydroxyethyl acrylate. VAc is vinyl acetate. | | | | | | |

| | | | | | | |
|---|---|---|---|---|---|---|
| *Compound A and Compound B are named in Example 1. | | | | | | |

In all the monomers except styrene, 10 ppm of Compound A or Compound B provides significantly greater polymerization inhibition compared to the unihibited controls.

### Example 4

Using the general method described in Example 3, the relative effectiveness of N-hydroxypiperidines is measured using methyl methacrylate monomer and 100 ppm of test inhibitor with the inhibited monomer being placed in sealed bottles at 80°C. The time in hours till gelation is visually observed is taken as a measure of the inhibition efficacy of the test compound.

| Inhibitor* | Hours to Failure (= Gelation) | |
|---|---|---|
| | N-hydroxy | N-oxyl |
| Compound A | >6900 | -- |
| Compound B | >2700 | -- |
| Compound C | 3190 | -- |
| Compound D | >8300 | -- |

| | | |
|---|---|---|
| *Compound A is N-(1-hydroxy-2,2,6,6-tetramethylpiperidin-4-yl)caprolactam. Compound B is bis(1-hydroxy-2,2,6,6-tetramethylpiperidin-4-yl) sebacate. Compound C is 1-hydroxy-2,2,6,6-tetramethylpiperidin-4-yl benzoate. Compound D is (1-hydroxy-2,2,6,6-tetramethylpiperidin-4-yl) acrylate. | | |

The data given in the table above show that the 1-hydroxy substituted piperidines are efficient in polymerization inhibition. Additionally, the 1-hydroxy compounds are essentially colorless.

### Example 5

Using the general procedure of Example 3 with commerical methyl methacrylate (MMA), with 10 ppm of monomethyl ether of hydroquinone present as an inhibitor, 100 ppm of test inhibitors are added to 25 g portions of the MMA in 28 ml bottles. The samples are placed in an oven at 80°C and the time required for polymerization or gelation to occur is visually observed.

| Inhibitor * | Hours to Failure (= Gelation) |
|---|---|
| Compound D | 1962 |
| Compound F | 370 |
| Compound G | 1400 |
| Compound H | 1000 |
| Compound I | 1400 |
| Compound J | 370 |
| Compound K | 1255 |
| Compound L | 250 |
| Compound M | 1335 |
| Compound N | 850 |
| Compound O | 880 |
| Compound D is (1-hydroxy-2,2,6,6-tetramethylpiperidin-4-yl) acrylate. Compound F is (1-hydroxy-2,2,6,6-tetramethylpiperidin-4-yl)3,5-di-tert-butyl-4-hydroxybenzoate. Compound G is 4,4'-ethylenebis(1-hydroxy-2,2,6,6-tetramethylpiperazin-3-one). Compound H is 1-hydroxy-2,2,6,6-tetramethylpiperidin-4-yl stearate. Compound I is bis(1-hydroxy-2,2,6,6-tetramethylpiperidin-4-yl) o-phthalate. Compound J is bis(1-hydroxy-2,2,6,6-tetramethylpiperidin-4-yl) 3,5-di-tert-butyl-4-hydroxybenzyl-butyl-malonate. Compound K is bis(1-hydroxy-2,2,6,6-tetramethylpiperidin-4-yl) n-butylmalonate. Compound L is 2,10-di-tert-butyl-4,8-dimethyl-6-(1-hydroxy-2,2,6,6-tetramethylpiperidin-4-yloxy)-dibenzo[d,g][1,3,2]-dioxaphosphocin. Compound M is 1-hydroxy-2,2,6,6-tetramethylpiperidin-4-yl 2-ethylhexanoate. Compound N is the dinonylnaphthalene disulfonic salt of bis(1-hydroxy-2,2,6,6-tetramethylpiperidin-4-yl) sebacate. Compound O is the phosphorous acid salt of bis(1-hydroxy-2,2,6,6-tetramethylpiperidin-4-yl) sebacate. | |

Each of these test inhibitors provides excellent polymerization inhibition protection to the methyl methacrylate monomer.

### Example 6

To assess the effect of the instant test inhibitors on color development in the inhibited monomer, 3 ml portions of methyl methacrylate containing 100 ppm of the test inhibitor are placed in clear sealable test tubes. The tubes are sealed and then heated at 80°C for 100 hours while protected from light. The tubes are then placed in a colorimeter and the yellowness index (YI) values of the samples are measured. The higher is the YI value the yellower and more discolored is the sample.

| Inhibitor* | Yellowness Index after 100 Hours |
|---|---|
| All samples | 33.9 (before heating at 80°C) |
| none | 42.4 (gelled in 24 hours) |
| Compound B | 41.7 |
| Compound B is bis(1-hydroxy-2,2,6,6-tetramethylpiperidin-4-yl) sebacate. | |

### Examples 7-8

### Vapor Phase Inhibition

To 105 g (100 ml) of acrylic acid is added 100 ppm by weight of the instant inhibitors. This acrylic acid solution is mixed well and then added to a resin kettle whose weight is known which is fitted with a reflux condenser and nitrogen inlet tube. A stream of nitrogen at 250 ml/min is used to flush the kettle for 15 minutes. The kettle containing the acrylic acid is then immersed into a 6-liter oil bath such that the top of the acrylic acid solution is approximately 5 cm) below the level of the oil surface. The kettle and its contents are heated at 150°C for 100 minutes causing the acrylic acid to reflux. White insoluble polymer, commonly called popcorn polymer, is observed to grow on the walls in the reflux region of the apparatus. The kettle is removed from the oil bath and cleaned free of oil. The kettle is rinsed with hexane to remove the acrylic acid monomer and to leave the polymer. The kettle and polymer are dried and weighed to determine the total amount of polymer collected on the inside wall of the kettle.

| Example | Inhibitor*(100 ppm) | Monomer**(100 ml) | Polymer Formed grams |
|---|---|---|---|
| 7 | Compound C | acrylic acid | 33 |
| 8 | Compound B (100) | acrylic acid | 56 |

| | | | |
|---|---|---|---|
| *Compound B is bis(1-hydroxy-2,2,6,6-tetramethylpiperidin-4-yl) sebacate. Compound C is (1-hydroxy-2,2,6,6-tetramethylpiperidin-4-yl) benzoate. | | | |
| **MMA is methyl methacrylate. HEMA is 2-hydroxyethyl methacrylate. HEA is 2-hydroxyethyl acrylate. | | | |

These data show that the instant compounds are effective in preventing the formation of popcorn polymer in the vapor phase.

## Claims

1. A monomer composition, stabilized against premature polymerization, which comprises
(a) an ethylenically unsaturated monomer or mixture of monomers, polymerizable by free radical initiation, and
(b) an effective amount, sufficient to inhibit the premature polymerization of component (a), of a compound or mixture of compounds of any of formula I to XIII, and salts thereof, 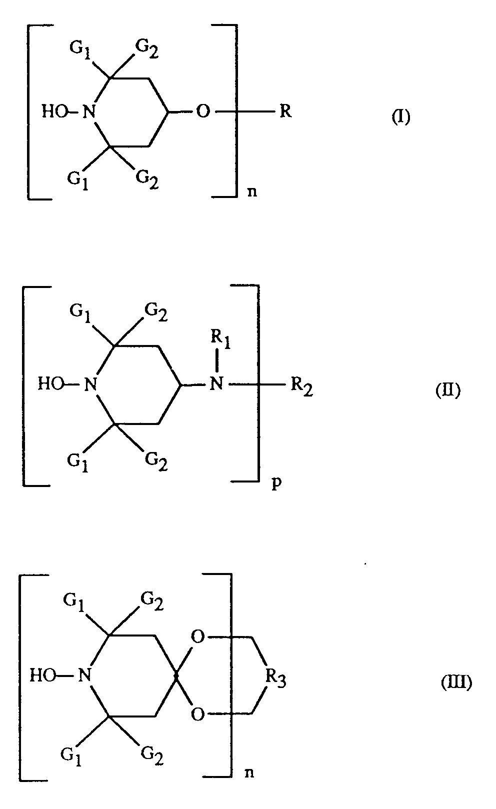 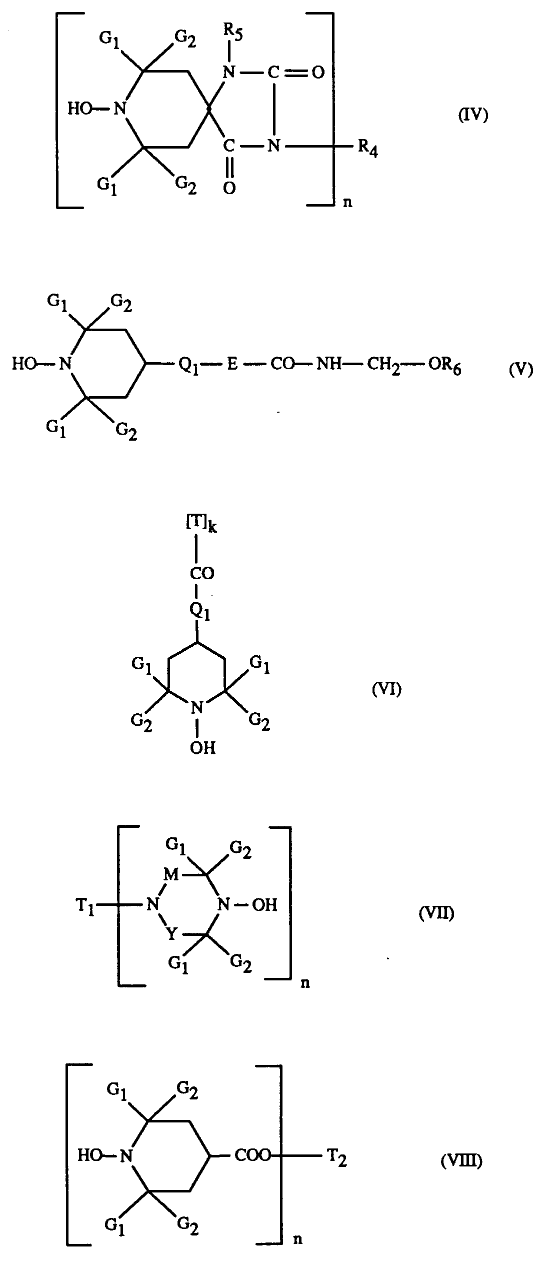 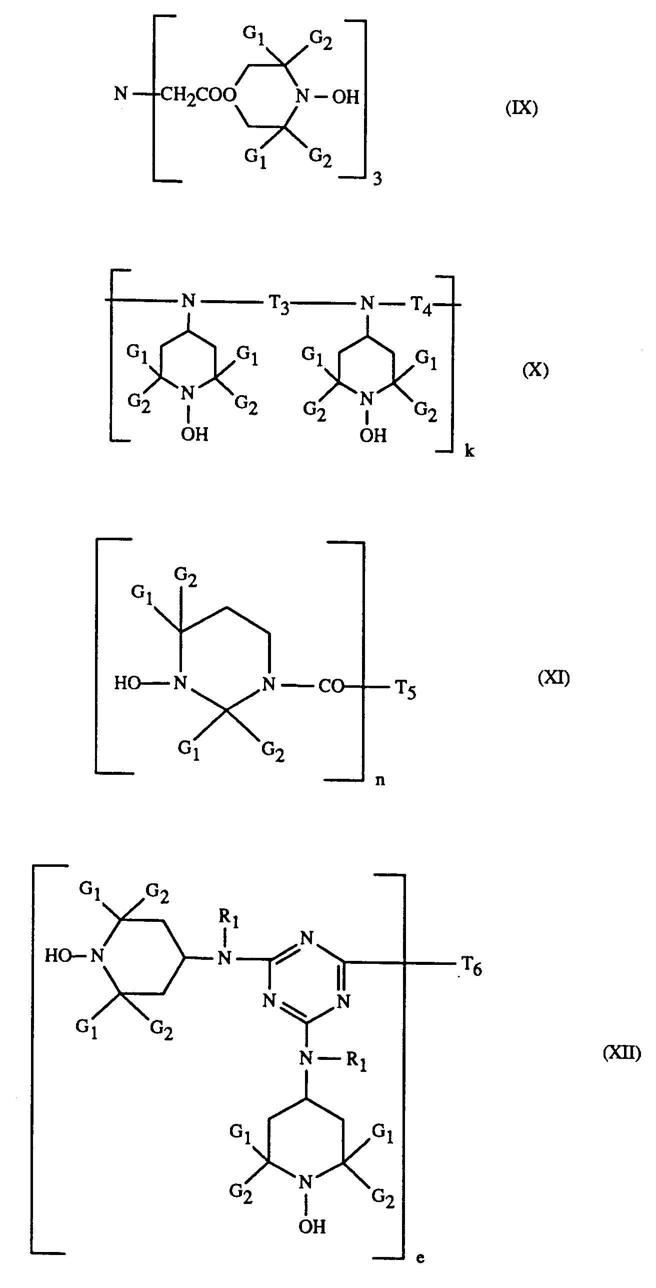 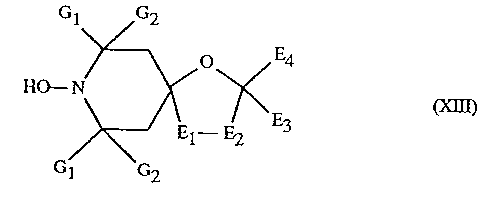
wherein
G₁ and G₂ are independently alkyl of 1 to 4 carbon atoms, or G₁ and G₂ together are pentamethylene;
n is 1 or 2,
when n is 1,
R is C₁-C₁₈-alkyl optionally interrupted by one or more oxygen atoms, cyanoethyl, benzyl, glycidyl, a monovalent acyl radical of an aliphatic, cycloaliphatic, araliphatic or aromatic acid, or of carbamic acid or of a phosphorus-containing acid, or a monovalent silyl radical; or
when n is 2,
R is C₁-C₁₂-alkylene, C₄-C₁₂-alkenylene, xylylene, a divalent acyl radical of an aliphatic, cycloaliphatic, araliphatic or aromatic dicarboxylic acid, or of a dicarbamic acid or of a phosphorus-containing acid, or a bivalent silyl radical;
p is 1, 2 or 3,
R₁ is hydrogen, C₁-C₁₂-alkyl, C₅-C₇-cycloalkyl, C₇-C₈-aralkyl, C₂-C₁₈-alkanoyl, C₃-C₅-alkenoyl or benzoyl;
when p is 1,
R₂ is hydrogen, C₁-C₁₈-alkyl, C₅-C₇-cycloalkyl, C₂-C₈-alkenyl unsubstituted or substituted by a cyano, carbonyl or carbamide group, or is glycidyl, a group of the formula -CH₂CH(OH)-Z or of the formula -CONH-Z wherein Z is hydrogen, methyl or phenyl; or
when p is 2,
R₂ is C₂-C₁₂-alkylene, C₆-C₁₂-arylene, xylylene, a -CH₂CH(OH)CH₂-O-X-O-CH₂CH(OH)CH₂- wherein X is C₂-C₁₀-alkylene, C₆-C₁₅-arylene or C₆-C₁₂-cycloalkylene; or, provided that R₁ is not alkanoyl, alkenoyl or benzoyl, R₂ can also be a divalent acyl radical of an aliphatic, cycloaliphatic or aromatic dicarboxylic acid or dicarbamic acid, or can be the group -CO-; or R₁ and R₂ together when p is 1 can be the cyclic acyl radical of an aliphatic or aromatic 1,2-or 1,3-dicarboxylic acid; or
R₂ is 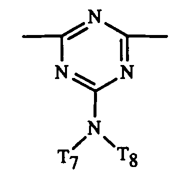 where T₇ and T₈ are independently hydrogen, alkyl of 1 to 18 carbon atoms, or T₇ and T₈ together are alkylene of 4 to 6 carbon atoms or 3-oxapentamethylene;
when p is 3,
R₂ is 2,4,6-triazinyl;
when n is 1,
R₃ is C₂-C₈-alkylene or hydroxyalkylene or C₄-C₂₂-acyloxyalkylene; or
when n is 2,
R₃ is (-CH₂)₂C(CH₂-)₂;
when n is 1,
R₄ is hydrogen, C₁-C₁₂-alkyl, C₃-C₅-alkenyl, C₇-C₉-aralkyl, C₅-C₇-cycloalkyl, C₂-C₄-hydroxyalkyl, C₂-C₆-alkoxyalkyl, C₆-C₁₀-aryl, glycidyl, a group of formula -(CH₂)ₘ-COO-Q or of the formula -(CH₂)ₘ-O-CO-Q wherein m is 1 or 2 and Q is C₁-C₄-alkyl or phenyl; or
when n is 2,
R₄ is C₂-C₁₂-alkylene, C₆-C₁₂-arylene, a group -CH₂CH(OH)CH₂-O-X-O-CH₂CH(OH)CH₂- wherein X is C₂-C₁₀-alkylene, C₆-C₁₅-arylene or C₆-C₁₂-cycloalkylene, or a group -CH₂CH(OZ₁)CH₂-(OCH₂CH(OZ₁)CH₂)₂- wherein Z₁ is hydrogen, C₁-C₁₈-alkyl, allyl, benzyl, C₂-C₁₂-alkanoyl or benzoyl;
R₅ is hydrogen, C₁-C₁₂-alkyl, allyl, benzyl, glycidyl or C₂-C₆-alkoxyalkyl;
Q₁ is -N(R₇)- or -O-;
E is C₁-C₃-alkylene, the group -CH₂CH(R₈)-O- wherein R₈ is hydrogen, methyl or phenyl, the group -(CH₂)₃-NH- or a direct bond;
R₇ is hydrogen, C₁-C₁₈-alkyl, C₅-C₇-cycloalkyl, C₇-C₁₂-aralkyl, cyanoethyl, C₆-C₁₀-aryl, the group -CH₂CH(R₈)-OH; or a group of the formula 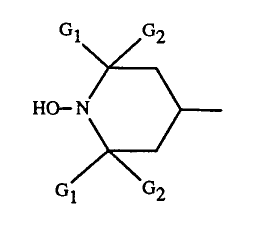 or a group of the formula 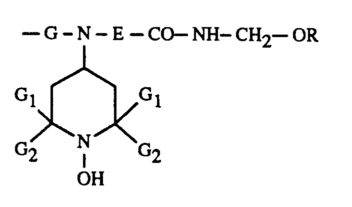 wherein G is C₂-C₆-alkylene or C₆-C₁₂-arylene; or
R₇ is a group -E-CO-NH-CH₂-OR₆;
R₆ is hydrogen or C₁-C₁₈-alkyl;
Formula VI denotes a recurring structural unit of a polymer where T is ethylene or 1,2-propylene, or is a repeating structural unit derived from an α-olefin copolymer with an alkyl acrylate or methacrylate;
k is 2 to 100;
T₁ has the same meaning as R₂ when p is 1 or 2;
M and Y are independently methylene or carbonyl;
T₂ has the same meaning as R₄;
T₃ and T₄ are independently alkylene of 2 to 12 carbon atoms, or T₄ is 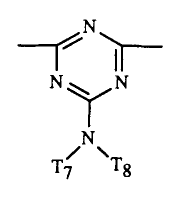 T₆ is 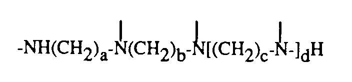 where a, b and c are independently 2 or 3, and d is 0 or 1;
e is 3 or 4;
T₅ is the same as R with the proviso that T₅ cannot be hydrogen when n is 1;
E₁ and E₂, being different, are each oxo or -N(E₅)- where E₅ is hydrogen, C₁-C₁₂-alkyl or alkoxycarbonylalkyl of 4 to 22 carbon atoms;
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms; and
E₄ is hydrogen, alkyl of 1 to 30 carbon atoms, phenyl, naphthyl or phenylalkyl of 7 to 12 carbon atoms; or
E₃ and E₄ together are polymethylene of 4 to 17 carbon atoms, or said polymethylene substituted by up to four alkyl groups of 1 to 4 carbon atoms.

2. A composition according to claim 1 wherein the effective amount of component (b) is 1 to 10000 ppm, preferably 5 to 2000 ppm and more preferably 50 to 1000 ppm, based on the total monomer composition.

3. A composition according to claim 1 wherein component (a) is a monomer selected from the group consisting of the olefinic hydrocarbons, dienes, halogenated monomers, unsaturated acids, unsaturated esters, unsaturated amides, unsaturated nitriles, unsaturated ethers, acrylated urethanes and unsaturated polyesters and mixtures thereof.

4. A composition according to claim 3 wherein the monomer is styrene, butadiene, vinyl chloride, acrylic acid, methacrylic acid, vinyl acetate, 2-hydroxyethyl acrylate, 2-hydroxyethyl methacrylate, trimethylolpropane triacrylate, polyethylene glycol diacrylate or methyl methacrylate.

5. A composition according to claim 4 wherein the monomer is styrene, butadiene, acrylic acid or methacrylic acid.

6. A composition according to claim 1 wherein component (b) is of formula I, II, or VII, preferably of formula I.

7. A composition according to claim 1 wherein component (b) is selected from the group consisting of
N-(1-hydroxy-2,2,6,6-tetramethylpiperidin-4-yl) caprolactam;
bis(1-hydroxy-2,2,6,6-tetramethylpiperidin-4-yl) sebacate;
1-hydroxy-2,2,6,6-tetramethylpiperidin-4-yl benzoate;
1-hydroxy-2,2,6,6-tetramethylpiperidin-4-yl acrylate;
1-hydroxy-2,2,6,6-tetramethylpiperidin-4-yl 3,5-di-tert-butyl-4-hydroxybenzoate;
4,4'-ethylenebis(1-hydroxy-2,2,6,6-tetramethylpiperazin-3-one);
1-hydroxy-2,2,6,6-tetramethylpiperidin-4-yl stearate;
bis(1-hydroxy-2,2,6,6-tetramethylpiperidin-4-yl) o-phthalate;
bis(1-hydroxy-2,2,6,6-tetramethylpiperidin-4-yl) 3,5-di-tert-butyl-4-hydroxybenzylbutyl-malonate;
bis(1-hydroxy-2,2,6,6-tetramethylpiperidin-4-yl) n-butylmalonate;
2,10-di-tert-butyl-4,8-dimethyl-6-(1-hydroxy-2,2,6,6-tetramethylpiperidin-4-yloxy)dibenzo[d,g][1,3,2]dioxaphosphocin;
1-hydroxy-2,2,6,6-tetramethylpiperidin-4-yl 2-ethylhexanoate;
dinonylnaphthalene disulfonic salt of bis(1-hydroxy-2,2,6,6-tetramethylpiperidin-4-yl) sebacate; and
the phosphorus acid salt of bis(1-hydroxy-2,2,6,6-tetramethylpiperidin-4-yl) sebacate.

8. A composition according to claim 1 which additionally contains another stabilizer selected from the group consisting of hydroquinone, monomethyl ether of hydroquinone and phenothiazine.

9. A process for preventing the premature polymerization of a monomer polymerizable by free radical initiation which comprises
adding to said monomer (a) an effective amount of a compound of component (b) according to claim 1.

10. A process according to claim 9 for preventing the fouling of processing equipment including reactors, pipes, stills, distillation columns, cracking towers and heat transfer surfaces during the processing of a monomer polymerizable by free radical intiation which comprises
adding to said monomer, before processing is begun, an effective amount of a compound of component (b) according to claim 1.

11. A process according to claim 9 which comprises
adding 10 to 500 ppm of at least one compound of formula I to XIII, according to claim 1, to a continuous fluid feed stream to deactivate the autocatalytic polymerization, in any part of the continuous process equipment, of any ethylenically unsaturated monomer present in the feed stream, and
further adding to said feed stream an additional 10 ppb to 500 ppm of at least one compound of formula I to XIII as a makeup additive to maintain the desired concentration of said compound in the fluid feed stream being processed.

## Patentansprüche

1. Monomerenzusammensetzung, die gegen vorzeitige Polymerisation stabilisiert ist, welche umfaßt
(a) ein ethylenisch ungesättigtes Monomer oder eine Mischung von Monomeren, das durch Radikalinitiierung polymerisierbar ist, und
(b) eine wirksame Menge, ausreichend um vorzeitige Polymerisation der Komponente (a) zu verhindern bzw. zu verzögern, einer Verbindung oder eines Gemisches von Verbindungen irgendeiner der Formeln I bis XIII und Salzen davon 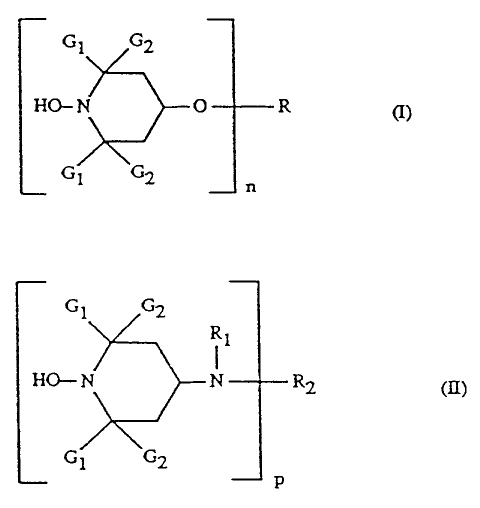 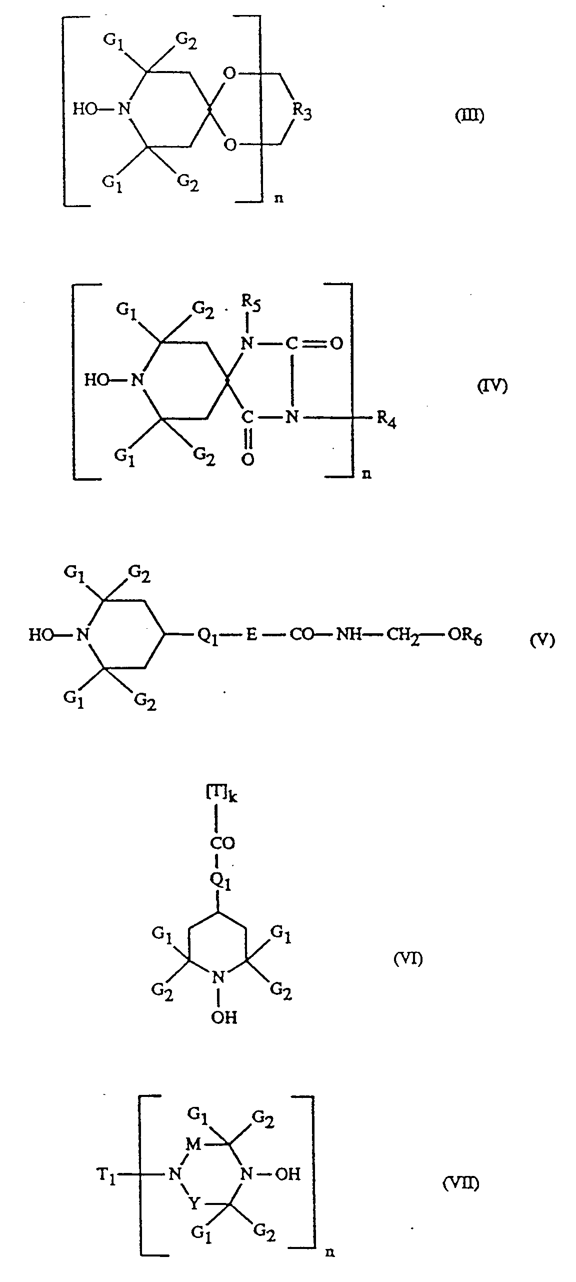 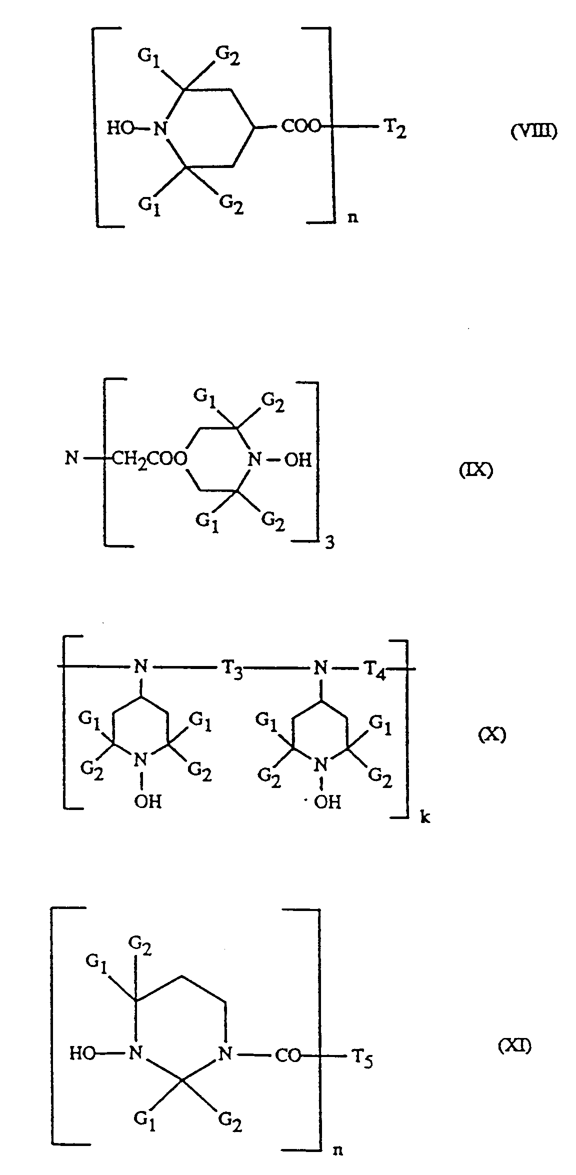 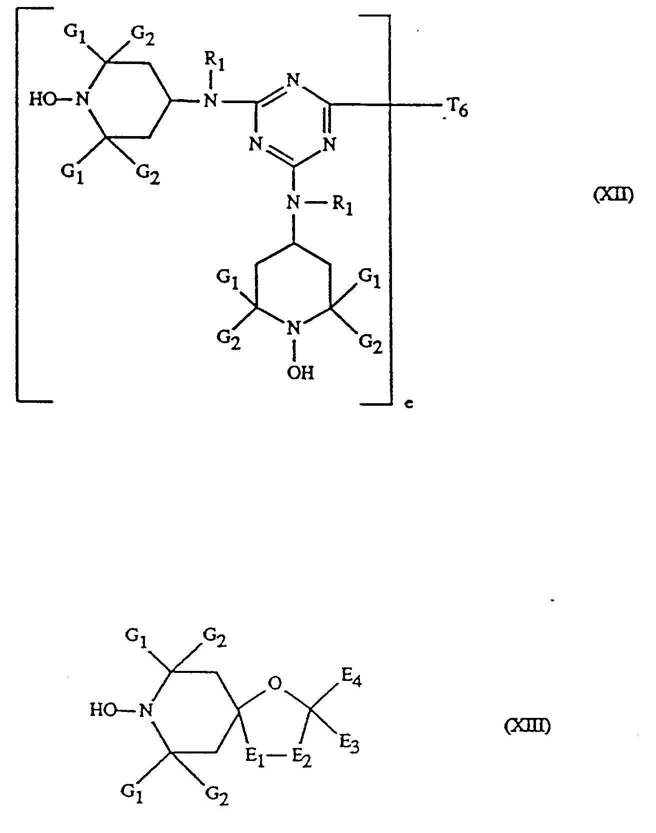
worin
G₁ und G₂ unabhängig Alkyl mit 1 bis 4 Kohlenstoffatomen sind, oder G₁ und G₂ sind zusammen Pentamethylen;
n ist 1 oder 2;
wenn n = 1 ist,
ist R C₁-C₁₈-Alkyl, gegebenenfalls unterbrochen durch ein oder mehrere Sauerstoffatom(e), Cyanoethyl, Benzyl, Glycidyl,
ein einwertiger Acylrest einer aliphatischen, cycloaliphatischen, araliphatischen oder aromatischen Säure, oder von Carbamidsäure oder einer phosphorhaltigen Säure, oder ein einwertiger Silylrest;oder
wenn n = 2 ist,
ist R C₁-C₁₂-Alkylen, C₄-C₁₂-Alkenylen, Xylylen, ein zweiwertiger Acylrest einer aliphatischen, cycloaliphatischen, araliphatischen oder aromatischen Dicarbonsäure, oder von einer Dicarbamidsäure oder einer phosphorhaltigen Säure, oder ein zweiwertiger Silylrest;
p ist 1, 2 oder 3,
R₁ ist Wasserstoff, C₁-C₁₂-Alkyl, C₅-C₇-Cycloalkyl, C₇-C₈-Aralkyl, C₂-C₁₈-Alkanoyl, C₃-C₅-Alkenoyl oder Benzoyl;
wenn p = 1 ist,
ist R₂ Wasserstoff, C₁-C₁₈-Alkyl, C₅-C₇-Cycloalkyl, C₂-C₈-Alkenyl unsubstituiert oder substituiert durch eine Cyano-, Carbonyl- oder Carbamidgruppe, oder ist Glycidyl, eine Gruppe der Formel -CH₂CH(OH)-Z oder der Formel -CONH-Z, worin Z Wasserstoff, Methyl oder Phenyl ist; oder
wenn p = 2 ist,
ist R₂ C₂-C₁₂-Alkylen, C₆-C₁₂-Arylen, Xylylen, ein Rest -CH₂CH(OH)CH₂-O-X-O-CH₂CH(OH)CH₂-, worin X C₂-C₁₀-Alkylen, C₆-C₁₅-Arylen oder C₆-C₁₂-Cycloalkylen
ist; oder, vorausgesetzt, daß R₁ nicht Alkanoyl, Alkenoyl oder Benzoyl ist, kann R₂ auch ein zweiwertiger Acylrest einer aliphatischen, cycloaliphatischen oder aromatischen Dicarbonsäure oder Dicarbamidsäure sein, oder kann die Gruppe -CO- sein; oder R₁ und R₂ können zusammen, wenn p = 1 ist, der cyclische Acylrest einer aliphatischen oder aromatischen 1,2- oder 1,3-Dicarbonsäure sein; oder
R₂ ist 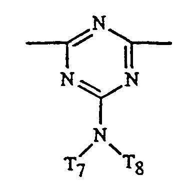 wobei T₇ und T₈ unabhängig Wasserstoff, Alkyl mit 1 bis 18 Kohlenstoffatomen sind, oder T₇ und T₈ sind zusammen Alkylen mit 4 bis 6 Kohlenstoffatomen oder 3-Oxapentamethylen;
wenn p = 3 ist,
ist R₂ 2,4,6-Triazinyl;
wenn n = 1 ist,
ist R₃ C₂-C₈-Alkylen oder Hydroxyalkylen oder C₄- C₂₂_Acyloxyalkylen; oder
wenn n = 2 ist,
ist R₃ (-CH₂)₂C(CH₂-)₂;
wenn n = 1 ist,
ist R₄ Wasserstoff, C₁-C₁₂-Alkyl, C₃-C₅-Alkenyl, C₇-C₉-Aralkyl, C₅-C₇-Cycloalkyl, C₂-C₄-Hydroxyalkyl, C₂-C₆-Alkoxyalkyl, C₆-C₁₀-Aryl, Glycidyl, eine Gruppe der Formel -(CH₂)ₘ-COO-Q oder der Formel -(CH₂)ₘ-O-CO-Q, worin m = 1 oder 2 ist und Q ist C₁-C₄-Alkyl oder Phenyl; oder
wenn n = 2 ist,
ist R₄ C₂-C₁₂-Alkylen, C₆-C₁₂-Arylen, eine Gruppe -CH₂CH(OH)CH₂-O-X-O-CH₂CH(OH)CH₂-, worin X C₂-C₁₀-Alkylen, C₆-C₁₅-Arylen oder C₆-C₁₂-Cycloalkylen bedeutet, oder eine Gruppe -CH₂CH(OZ₁)CH₂(OCH₂CH(OZ₁)CH₂)₂-, worin Z₁ Wasserstoff, C₁-C₁₈-Alkyl, Allyl, Benzyl, C₂-C₁₂-Alkanoyl oder Benzoyl bedeutet,
R₅ ist Wasserstoff, C₁-C₁₂-Alkyl, Allyl, Benzyl, Glycidyl oder C₂-C₆-Alkoxyalkyl;
Q₁ ist -N(R₇)- oder -O-;
E ist C₁-C₃-Alkylen, die Gruppe -CH₂CH(R₈)-O-, worin R₈ Wasserstoff, Methyl oder Phenyl ist, die Gruppe -(CH₂)₃NH- oder eine direkte Bindung;
R₇ ist Wasserstoff, C₁-C₁₈-Alkyl, C₅-C₇-Cycloalkyl, C₇-C₁₂-Aralkyl, Cyanoethyl, C₆-C₁₀-Aryl, die Gruppe -CH₂CH(R₈)-OH; oder eine Gruppe der Formel 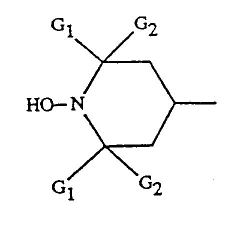 oder eine Gruppe der Formel 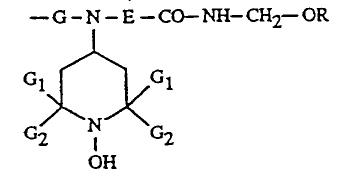 worin G C₂-C₆-Alkylen oder C₆-C₁₂-Arylen ist; oder
R₇ ist eine Gruppe -E-CO-NH-CH₂-OR₆;
R₆ ist Wasserstoff oder C₁-C₁₈-Alkyl;
die Formel VI bezeichnet eine wiederkehrende Struktureinheit eines Polymeren, worin T Ethylen oder 1,2-Propylen ist, oder ist eine wiederkehrende Struktureinheit, abgeleitet von einem α-Olefincopolymeren mit einem Alkylacrylat oder -methacrylat;
k ist 2 bis 100;
T₁ hat die gleiche Bedeutung wie R₂, wenn p = 1 oder 2 ist;
M und Y sind unabhängig Methylen oder Carbonyl;
T₂ hat die gleiche Bedeutung wie R₄;
T₃ und T₄ sind unabhängig Alkylen mit 2 bis 12 Kohlenstoffatomen, oder T₄ ist 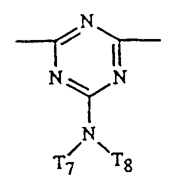 T₆ ist 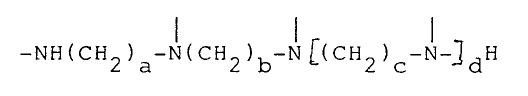 worin a, b und c unabhängig 2 oder 3 sind, und d ist 0 oder 1;
e ist 3 oder 4;
T₅ ist dasselbe wie R mit der Maßgabe, daß T₅ nicht Wasserstoff sein kann, wenn n = 1 ist;
E₁ und E₂, die verschieden sind, sind jeweils Oxo oder -N(E₅), worin E₅ Wasserstoff ist, C₁-C₁₂-Alkyl oder Alkoxycarbonylalkyl mit 4 bis 22 Kohlenstoffatomen;
E₃ ist Wasserstoff, Alkyl mit 1 bis 30 Kohlenstoffatomen, Phenyl, Naphthyl, wobei das genannte Phenyl oder das genannte Naphthyl substituiert ist durch Chlor oder durch Alkyl mit 1 bis 4 Kohlenstoffatomen, oder Phenylalkyl mit 7 bis 12 Kohlenstoffatomen, oder dieses Phenylalkyl substituiert durch Alkyl mit 1 bis 4 Kohlenstoffatomen; und
E₄ ist Wasserstoff, Alkyl mit 1 bis 30 Kohlenstoffatomen, Phenyl, Naphthyl oder Phenylalkyl mit 7 bis 12 Kohlenstoffatomen; oder
E₃ und E₄ sind zusammen Polymethylen mit 4 bis 17 Kohlenstoffatomen, oder dieses Polymethylen substituiert mit bis zu 4 Alkylgruppen mit 1 bis 4 Kohlenstoffatomen.

2. Zusammensetzung gemäß Anspruch 1, worin die wirksame Menge der Komponente (b) 1 bis 10000 ppm, vorzugsweise 5 bis 2000 ppm, und noch bevorzugter 50 bis 1000 ppm auf Basis der Gesamtmonomerenzusammensetzung beträgt.

3. Zusammensetzung gemäß Anspruch 1, worin die Komponente (a) ein Monomeres ist, ausgewählt aus der Gruppe bestehend aus den olefinischen Kohlenwasserstoffen, Dienen, halogenierten Monomeren, ungesättigten Säuren, ungesättigten Estern, ungesättigten Amiden, ungesättigten Nitrilen, ungesättigten Ethern, acrylierten Urethanen und ungesättigten Polyestern und Mischungen davon.

4. Zusammensetzung gemäß Anspruch 3, worin das Monomere Styrol, Butadien, Vinylchlorid, Acrylsäure, Methacrylsäure, Vinylacetat, 2-Hydroxyethylacrylat, 2-Hydroxyethylmethacrylat, Trimethylolpropantriacrylat, Polyethylenglykoldiacrylat oder Methylmethacrylat ist.

5. Zusammensetzung gemäß Anspruch 4, worin das Monomere Styrol, Butadien, Acrylsäure oder Methacrylsäure ist.

6. Zusammensetzung gemäß Anspruch 1, worin die Komponente (b) der Formel I, II oder VII entspricht, vorzugsweise der Formel I.

7. Zusammensetzung gemäß Anspruch 1, worin die Komponente (b) ausgewählt ist aus der Gruppe bestehend aus
N-(1-Hydroxy-2,2,6,6-tetramethylpiperidin-4-yl)-caprolactam;
Bis-(1-hydroxy-2,2,6,6-tetramethylpiperidin-4-yl)-sebacat;
1-Hydroxy-2,2,6,6-tetramethylpiperidin-4-yl-benzoat;
1-Hydroxy-2,2,6,6-tetramethylpiperidin-4-yl-acrylat;
1-Hydroxy-2,2,6,6-tetramethylpiperidin-4-yl-3,5-di-tert-butyl-4-hydroxybenzoat;
4,4'-Ethylenbis-(1-hydroxy-2,2,6,6-tetramethylpiperazin-3-on);
1-Hydroxy-2,2,6,6-tetramethylpiperidin-4-yl-stearat;
Bis-(1-hydroxy-2,2,6,6-tetramethylpiperidin-4-yl)-o-phthalat;
Bis-(1-hydroxy-2,2,6,6-tetramethylpiperidin-4-yl)-3,5-di-tert-butyl-4-hydroxybenzylbutyl-malonat;
Bis-(1-hydroxy-2,2,6,6-tetramethylpiperidin-4-yl)-n-butylmalonat;
2,10-Di-tert-butyl-4,8-dimethyl-6-(1-hydroxy-2,2,6,6-tetramethylpiperidin-4-yl-oxy)-dibenzo[d,g][1,3,2]dioxaphosphocin;
1-Hydroxy-2,2,6,6-tetramethylpiperidin-4-yl-2-ethylhexanoat;
das Dinonylnaphthalindisulfonsäuresalz von Bis-(1-hydroxy-2,2,6,6-tetramethylpiperidin-4-yl)-sebacat; und
das phosphorsaure Salz von Bis-(1-hydroxy-2,2,6,6-tetramethylpiperidin-4-yl)-sebacat.

8. Zusammensetzung gemäß Anspruch 1, die außerdem einen weiteren Stabilisator ausgewählt aus der Gruppe bestehend aus Hydrochinon, Monomethylether von Hydrochinon und Phenothiazin enthält.

9. Verfahren zur Verhinderung bzw. Vermeidung der vorzeitigen Polymerisation eines Monomeren, das durch Radikalinitiierung polymerisierbar ist, welches umfaßt
die Zugabe einer wirksamen Menge einer Verbindung der Komponente (b) gemäß Anspruch 1 zu diesem Monomeren (a).

10. Verfahren gemäß Anspruch 9 zur Vorbeugung bzw. Verhinderung der Verschmutzung von Verfahrenseinrichtungen einschließlich Reaktoren, Röhren bzw. Rohrleitungen, Destillationsapparaten, Destillationskolonnen, Cracktürmen und Wärmeübertragungsflächen während der Verarbeitung eines Monomeren, das durch Radikalinitiierung polymerisierbar ist, welches umfaßt
die Zugabe einer wirksamen Menge einer Verbindung der Komponente (b) gemäß Anspruch 1 zu diesem Monomeren, bevor das Verfahren bzw. die Verarbeitung beginnt.

11. Verfahren gemäß Anspruch 9, welches umfaßt
die Zugabe von 10 bis 500 ppm mindestens einer Verbindung der Formel I bis XIII gemäß Anspruch 1 zu einem kontinuierlichen fluiden Speisungsstrom zur Desaktivierung der autokatalytischen Polymerisation in irgendeinem Teil der Apparatur des kontinuierlichen Verfahrens, von irgendeinem ethylenisch ungesättigten Monomeren, das in dem Speisungsstrom vorhanden ist, und
weiterhin die Zugabe zu diesem Strom von zusätzlichen 10 ppb bis 500 ppm von mindestens einer Verbindung der Formel I bis XIII als Auffüllungszusatz, um die gewünschte Konzentration dieser Verbindung in dem der Verarbeitung unterworfenen fluiden Speisungsstrom aufrechtzuerhalten.

## Revendications

1. Une composition de monomère, stabilisée contre une polymérisation prématurée, qui comprend
(a) un monomère ou un mélange de monomères éthyléniquement insaturés, polymérisables par initiation radicalaire, et
(b) une quantité efficace, suffisante pour inhiber la polymérisation prématurée du composant (a), d'un composé ou mélange de composés de l'une quelconque des formules I a XIII, ou de leurs sels, 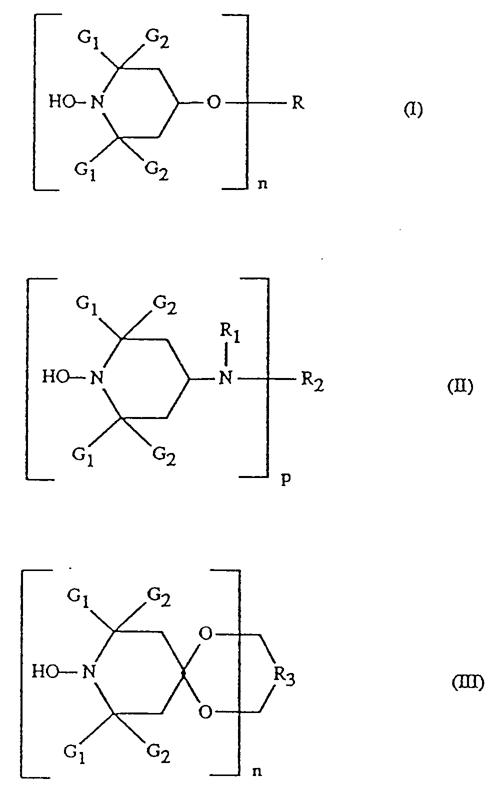 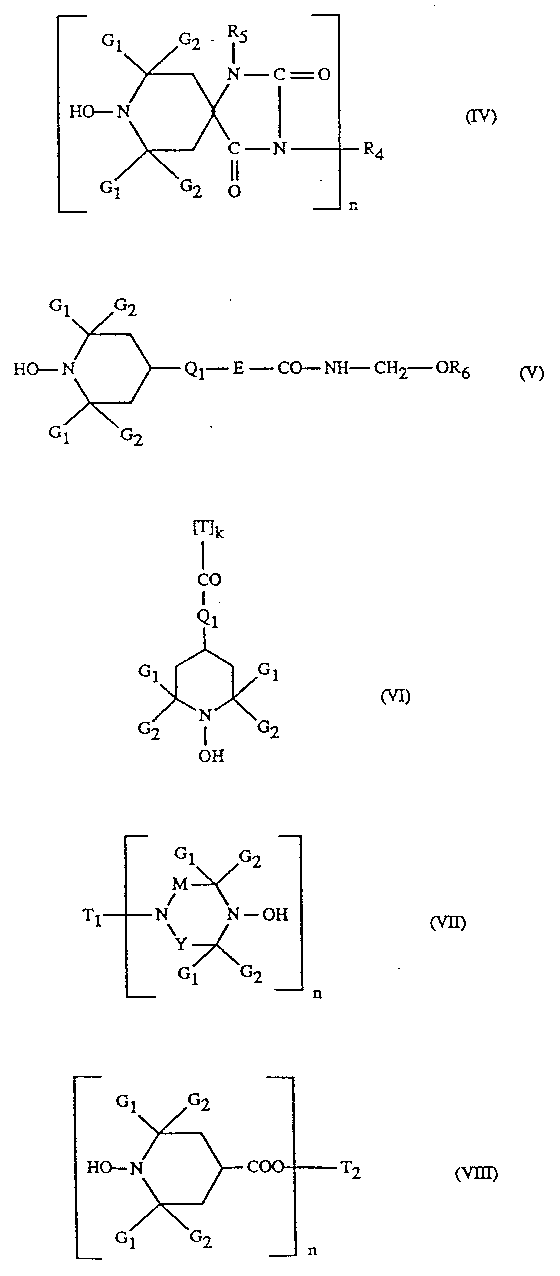 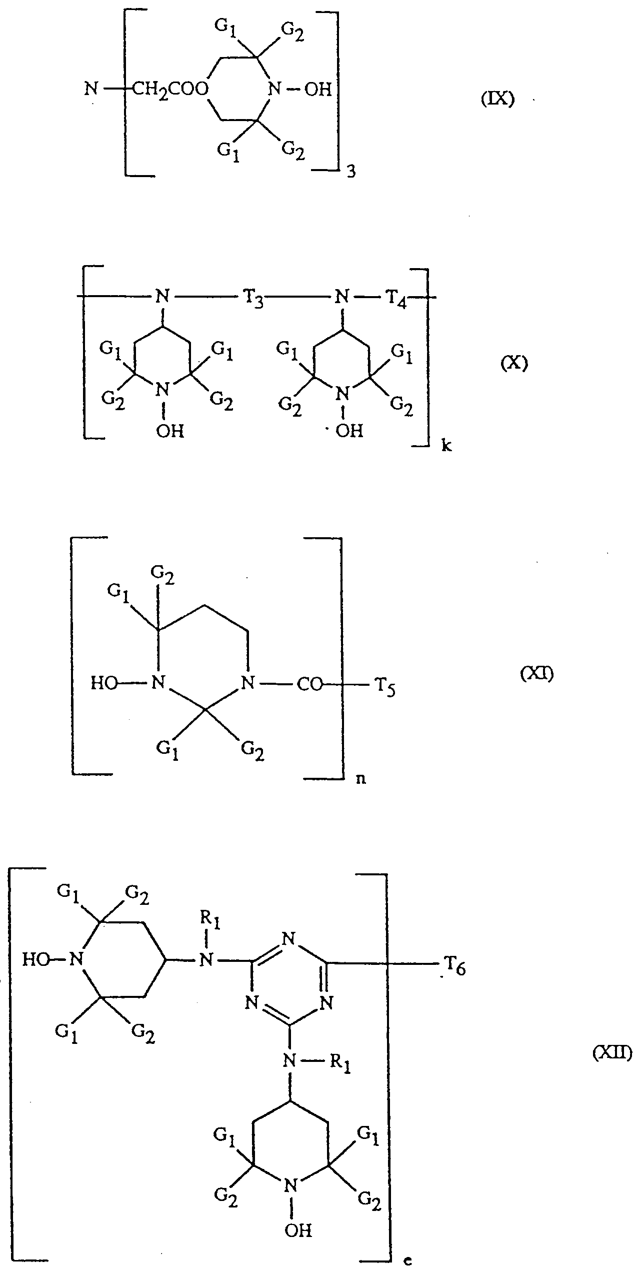 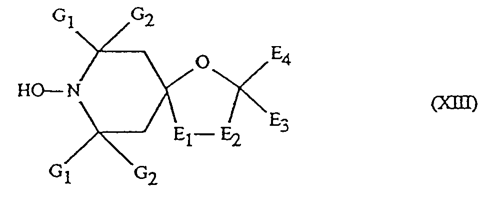
où
G₁ et G₂ sont chacun indépendamment un groupe alkyle de 1 à 4 atomes de carbone, ou bien G₁ et G₂ sont ensemble un groupe pentaméthylène ;
n est 1 ou 2,
lorsque n est 1,
R est un groupe alkyle en C₁-C₁₈ facultativement interrompu par un ou plusieurs atomes d'oxygène, un groupe cyanoéthyle, un groupe benzyle, un groupe glycidyle, un radical acyle monovalent d'un acide aliphatique, cycloaliphatique, araliphatique ou aromatique ou d'acide carbamique ou d'un acide phosphoré, ou un radical silyle monovalent ; ou
lorsque n est 2,
R est un groupe alkylène en C₁-C₁₂, un groupe alcénylène en C₄-C₁₂, un groupe xylylène, un radical acyle divalent d'un acide dicarboxylique aliphatique, cycloaliphatique, araliphatique ou aromatique, ou d un acide dicarbamique, ou d'un acide phosphoré, ou un radical silyle divalent ;
p est 1, 2 ou 3,
R₁ est l'hydrogène, un groupe alkyle en C₁-C₁₂, cycloalkyle en C₅-C₇, aralkyle en C₇-C₈, alcanoyle en C₂-C₁₈, alcénoyle en C₃-C₅ ou benzoyle ;
lorsque p est 1,
R₂ est l'hydrogène, un groupe alkyle en C₁-C₁₈, un groupe cycloalkyle en C₅-C₇, un groupe alcényle en C₂-C₈ non substitué ou substitué par un groupe cyano, carbonyle ou carbamide, ou est un groupe glycidyle ou un groupe de formule -CH₂CH(OH)-Z ou de formule -CONH-Z où Z est l'hydrogène, un groupe méthyle ou phényle ; ou
lorsque p est 2,
R₂ est un groupe alkylène en C₂-C₁₂, un groupe arylène en C₆-C₁₂, un groupe xylylène, un groupe -CH₂CH(OH)CH₂-O-X-O-CH₂CH(OH)CH₂- où X est un groupe alkylène en C₂-C₁₀, arylène en C₆-C₁₅ ou cycloalkylène en C₆-C₁₂ ; ou, si R₁ n'est pas un groupe alcanoyle, alcénoyle ou benzoyle, R₂ peut également être un radical acyle divalent d'un acide dicarboxylique aliphatique, cycloaliphatique ou aromatique ou d'acide dicarbamique, ou peut être le groupe -CO- ; ou bien, lorsque p est 1, R₁ et R₂ peuvent former ensemble le radical acyle cyclique d'un acide 1,2- ou 1,3-dicarboxylique aliphatique ou aromatique ; ou
R₂ est 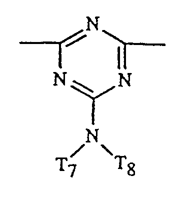 où T₇ et T₈ sont chacun indépendamment l'hydrogène, un groupe alkyle de 1 à 18 atomes de carbone, ou bien T₇ et T₈ forment ensemble un groupe alkylène de 4 à 6 atomes de carbone ou 3-oxapentaméthylène ;
lorsque p est 3,
R₂ est un groupe 2,4,6-triazinyle ;
lorsque n est 1,
R₃ est un groupe alkylène ou hydroxyalkylène en C₂-C₈ ou acyloxyalkylène en C₄-C₂₂ ; ou
lorsque n est 2,
R₃ est (-CH₂)₂C(CH₂-)₂ ;
lorsque n est 1,
R₄ est l'hydrogène, un groupe alkyle en C₁-C₁₂, alcényle en C₃-C₅, aralkyle en C₇-C₉, cycloalkyle en C₅-C₇, hydroxyalkyle en C₂-C₄, alcoxyalkyle en C₂-C₆, aryle en C₆-C₁₀, glycidyle, un groupe de formule -(CH₂)ₘ-COO-Q ou de formule -(CH₂)ₘ-O-CO-Q où m est 1 ou 2 et Q est un groupe alkyle en C₁-C₄ ou phényle ; ou
lorsque n est 2,
R₄ est un groupe alkylène en C₂-C₁₂, un groupe arylène en C₆-C₁₂, un groupe -CH₂CH(OH)CH₂-O-X-O-CH₂CH(OH)CH₂- où X est un groupe alkylène en C₂-C₁₀, arylène en C₆-C₁₅ ou cycloalkylene en C₆-C₁₂, ou un groupe -CH₂CH(OZ₁)CH₂-(OCH₂CH(OZ₁)CH₂)₂- où Z₁ est l'hydrogène, un groupe alkyle en C₁-C₁₈, allyle, benzyle, alcanoyle en C₂-C₁₂ ou benzoyle ;
R₅ est l'hydrogène, un groupe alkyle en C₁-C₁₂, allyle, benzyle, glycidyle ou alcoxyalkyle en C₂-C₆ ;
Q₁ est -N(R₇)- ou -O- ;
E est un groupe alkylène en C₁-C₃, un groupe -CH₂CH(R₈)-O- où R₈ est l'hydrogène, un groupe méthyle ou phényle, le groupe -(CH₂)₃-NH- ou une liaison directe ;
R₇ est l'hydrogène, un groupe alkyle en C₁-C₁₈, cycloalkyle en C₅-C₇, aralkyle en C₇-C₁₂, cyanoéthyle, aryle en C₆-C₁₀, un groupe -CH₂CH(R₈)-OH ou un groupe de formule 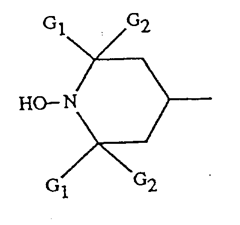 ou un groupe de formule 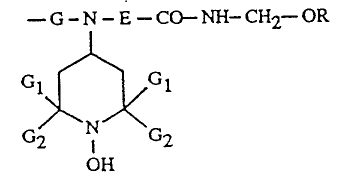 où G est un groupe alkylène en C₂-C₆ ou arylène en C₆-C₁₂ ; ou bien
R₇ est un groupe -E-CO-NH-CH₂-OR₆ ;
R₆ est l'hydrogène ou un groupe alkyle en C₁-C₁₈ ;
la Formule VI représente un motif structural récurrent d'un polymère dans lequel T est un groupe éthylène ou 1,2-propylène, ou est un motif structural récurrent dérivé d'un copolymère de α-oléfine avec un acrylate ou méthacrylate d'alkyle ;
k est de 2 à 100 ;
T₁ a la même signification que R₂ lorsque p est 1 ou 2 ;
M et Y sont chacun indépendamment un groupe méthylène ou carbonyle ;
T₂ a la même signification que R₄ ;
T₃ et T₄ sont chacun indépendamment un groupe alkylène de 2 à 12 atomes de carbone, ou bien T₄ est 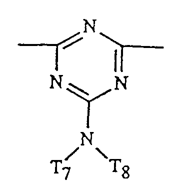 T₆ est 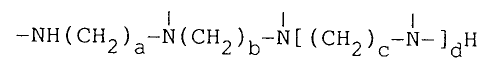 où a, b et c sont chacun indépendamment 2 ou 3 et d est 0 ou 1 ;
e est 3 ou 4 ;
T₅ est la même chose que R, à condition que T₅ ne soit pas l'hydrogène lorsque n est 1 ;
E₁ et E₂, qui sont différents, sont chacun un groupe oxo ou -N(E₅)- où E₅ est l'hydrogène, un groupe alkyle en C₁-C₁₂ ou alcoxycarbonylalkyle de 4 à 22 atomes de carbone ;
E₃ est l'hydrogène, un groupe alkyle de 1 à 30 atomes de carbone, un groupe phényle, un groupe naphtyle, ledit groupe phényle ou ledit groupe naphtyle substitué par du chlore ou par un groupe alkyle de 1 à 4 atomes de carbone, ou est un groupe phénylalkyle de 7 à 12 atomes de carbone ou un tel groupe phénylalkyle substitué par un groupe alkyle de 1 à 4 atomes de carbone ; et
E₄ est l'hydrogène, un groupe alkyle de 1 à 30 atomes de carbone, phényle, naphtyle ou phénylalkyle de 7 à 12 atomes de carbone ; ou bien
E₃ et E₄ forment ensemble un groupe polyméthylène de 4 à 17 atomes de carbone, ou un tel groupe polyméthylène substitué par un maximum de quatre groupes alkyle de 1 à 4 atomes de carbone.

2. Une composition selon la revendication 1, dans laquelle la quantité efficace de composant (b) est de 1 à 10 000 ppm, de préférence 5 à 2000 ppm, et mieux encore 50 à 1000 ppm,, par rapport à la composition de monomère totale.

3. Une composition selon la revendication 1, dans laquelle le composant (a) est un monomère choisi dans le groupe formé par les hydrocarbures oléfiniques, les diènes, les monomères halogénés, les acides insaturés, les esters insaturés, les amides insaturés, les nitriles insaturés, les éthers insaturés, les uréthannes acrylés et les polyesters insaturés, et leurs mélanges.

4. Une composition selon la revendication 3, dans laquelle le monomère est le styrène, le butadiène, le chlorure de vinyle, l'acide acrylique, l'acide méthacrylique, l'acétate de vinyle, l'acrylate de 2-hydroxyéthyle, le méthacrylate de 2-hydroxyéthyle, le triacrylate de triméthylolpropane, un diacrylate de polyéthylène-glycol ou le méthacrylate de méthyle.

5. Une composition selon la revendication 4, dans laquelle le monomère est le styrène, le butadiène, l'acide acrylique ou l'acide méthacrylique.

6. Une composition selon la revendication 1, dans laquelle le composant (b) répond à la formule I, II ou VII, de préférence la formule I.

7. Une composition selon la revendication 1, dans laquelle le composant (b) est choisi dans le groupe formé par
le N-(1-hydroxy-2,2,6,6-tétraméthylpipéridine-4-yl)-caprolactame ;
le sébacate de bis(1-hydroxy-2,2,6,6-tétraméthylpipéridine-4-yle) ;
le benzoate de 1-hydroxy-2,2,6,6-tétraméthylpipéridine-4-yle ;
l'acrylate de 1-hydroxy-2,2,6,6-tétraméthylpipéridine-4-yle ;
le 3,5-di-*tert*-butyl-4-hydroxybenzoate de 1-hydroxy-2,2,6,6-tétraméthylpipéridine-4-yle ;
le4,4'-éthylène-bis(1-hydroxy-2,2,6,6-tétraméthylpipérazine-3-one) ;
le stéarate de 1-hydroxy-2,2,6,6-tétraméthylpipéridine-4-yle ;
le *o*-phtalate de bis(1-hydroxy-2,2,6,6-tétraméthylpipéridine-4-yle) ;
le 3,5-di-*tert*-butyl-4-hydroxybenzyl-butylmalonate de bis(1-hydroxy-2,2,6,6-tétraméthylpipéridine-4-yle) ;
le *n*-butylmalonate de bis(1-hydroxy-2,2,6,6-tétraméthylpipéridine-4-yle) ;
le 2,10-di-*tert*-butyl-4,8-diméthyl-6-(1-hydroxy-2,2,6,6-tétraméthylpipéridine-4-yloxy)dibenzo[d,g][1,3,2]-dioxaphosphocine ;
le 2-éthylhexanoate de 1-hydroxy-2,2,6,6-tétraméthylpipéridine-4-yle ;
un sel d'acide dinonylnaphtalène-disulfonique de sébacate de bis(1-hydroxy-2,2,6,6-tétraméthylpipéridine-4-yle) ; et
le sel d'acide phosphoreux de sébacate de bis(1-hydroxy-2,2,6,6-tétraméthylpipéridine-4-yle).

8. Une composition selon la revendication 1, qui contient de plus un autre stabilisant choisi dans le groupe formé par l'hydroquinone, l'éther monométhylique d'hydroquinone et la phénothiazine.

9. Un procédé pour empêcher la polymérisation prématurée d'un monomère polymérisable par initiation radicalaire, qui comprend
l'addition audit monomère (a) d'une quantité efficace d'un composé du composant (b) selon la revendication 1.

10. Un procédé selon la revendication 9 pour empêcher l'encrassement d'appareils de traitement comprenant des réacteurs, conduites, appareils de distillation, colonnes de distillation, tours de craquage et surfaces de transfert de chaleur, pendant le traitement d'un monomère polymérisable par initiation radicalaire, qui comprend
l'addition audit au monomère, avant le début du traitement, d une quantité efficace d'un composé du composant (b) selon la revendication 1.

11. Un procédé selon la revendication 9, qui comprend
l'addition de 10 à 500 ppm d'au moins un composé de formule I à XIII, selon la revendication 1, à un courant d'alimentation continu de fluide, en toute partie de l'appareillage de traitement continu, pour désactiver la polymérisation autocatalytique de tout monomère éthyléniquement insaturé présent dans le courant d'alimentation, et
l'addition supplémentaire audit courant d'alimentation d'un supplément de 0,01 ppm à 500 ppm d'au moins un composé de formule I à XIII comme additif d'appoint pour maintenir la concentration désirée dudit composé dans le courant d'alimentation de fluide soumis au traitement.
